# EUROPEAN PATENT SPECIFICATION

(11) **EP 1 856 453 B1**
(45) Date of publication and mention of the grant of the patent: **13.07.2016**
(21) Application number: 06737726.7
(22) Date of filing: 10.03.2006
(51) Int. Cl.: F24F 11/00, G01F 23/00

(54) **DYNAMIC CONTROL OF DILUTION VENTILATION IN ONE-PASS, CRITICAL ENVIRONMENTS**
DYNAMISCHE STEUERUNG VON VERDÜNNUNGSBELÜFTUNG IN KRITISCHEN EINPASSUMGEBUNGEN
REGULATION DYNAMIQUE DE VENTILATION A DILUTION DANS DES ENVIRONNEMENTS CRITIQUES MONO-PASSE

(30) Priority: 10.03.2005 US 660245 P
(43) Date of publication of application: 21.11.2007
(73) Proprietor: Aircuity Incorporated, Newton, MA 02458 (US)
(72) Inventor: DESROCHERS, Eric, M., Millis, MA 02054 (US); SHARP, Gordon, P., Newton, MA 02468 (US)
(74) Representative: Jackson, Richard Eric
(86) International application number: PCT/US2006/008575
(87) International publication number: WO 2006/099125

(56) References cited:
- US-A- 4 049 404
- US-A- 5 431 599
- US-A- 5 544 809
- US-A1- 2002 072 322
- US-B1- 6 369 716
- US-B1- 6 711 470

## Description

### FIELD OF THE INVENTION

This invention relates to systems for controlling ventilation to dilute contaminants within critical environments or spaces such as laboratories and vivariums which utilize a "one-pass" ventilation strategy in which the airflow out of each environment is entirely exhausted without a recirculated air component, and more particularly, to systems and methods for varying the flows of supply and exhaust air into and from these environments for the purposes of controlling the dilution of air contaminants based on changes in the presence of these contaminants as sensed by a facility monitoring system.

### BACKGROUND OF THE INVENTION

This invention relates to the dynamic control of dilution ventilation in one-pass, critical environments. Critical environments in the context of this invention relate to spaces, areas or rooms in which potentially hazardous materials may be used that could become airborne and impact in some way the health of individuals operating within the space. Examples of such spaces include but are not limited to laboratories where chemicals or biological materials are used as well as vivariums or animal research facilities where animals are housed for research purposes. Other types of applicable areas include but are not limited to: clean rooms, pharmaceutical processing areas, bio-safety facilities, and medical containment and isolation facilities. Furthermore, critical environments may be further narrowed down to those which are strictly one-pass environments, which in the context of this invention refers to those spaces which use one-pass air, in other words no air from the space is returned or recirculated to an air handler or fan system for use again within the building. As such all air introduced to within the space either from a supply system, or as transfer air from another space, is exhausted typically through some sort of exhaust system which takes the room air and exhausts it from the building typically controlled by some sort of room exhaust or special exhaust air flow control device. Specifically, these rooms have no return air grills or return airflow control devices. Control of the room's airflow is thus accomplished through one or more exhaust airflow control devices designated for the space plus one or more supply airflow control devices that although typically are designated for the space, may also be somewhat remote from the room if some of the air from those devices enters the space as transfer air from another space that is provided with supply airflow via a separate air flow control device. Furthermore, a flow of air that is drawn from or supplied to a space or environment may be expressed as a volume of air per unit time, for example, in terms of cubic feet per minute (cfm).

Dilution ventilation as used in the context of this invention refers to the use of airflow supplied to the room either directly or as transfer air that dilutes or reduces the concentration of possible contaminants in the air of the room. Although capture and containment of hazardous vapors is the safest approach to handling hazardous materials, dilution ventilation provides an important backup or secondary form of protection for the critical environment in case the primary or containment control device malfunctions, or else an accident or spill occurs, or the occupants of the space use unsafe practices that introduce contaminants into the air of the room. Dilution ventilation is also sometimes referred to or defined as the minimum level of airflow allowed in the room, or as the airflow corresponding to the critical environment's minimum air change requirement typically expressed as a minimum air changes per hour (ACH) for the space.

As the price of oil, natural gas and other fuel sources has increased over the years, there has been interest in reducing the amount of outside air that is used by buildings to save energy, while still maintaining good indoor environmental quality within those facilities. In the context of this invention outside air is defined as the ambient air outside of the building housing the critical environment that maybe drawn into the building to provide some measure of fresh air ventilation. Since laboratory and vivarium facilities typically use 100% outside air these facilities use extremely large amounts of energy compared to other types of facilities such as office buildings that are able to use return or recirculated air. The purpose of the present invention is to significantly reduce the energy consumption of a critical environment utilizing one-pass or 100% exhaust air for dilution and potentially other purposes while also enhancing the safety of these environments.

As mentioned earlier, one example of a critical environment in which a one-pass, dilution ventilation system may be employed is a laboratory. A laboratory generally is a facility that is designed to permit the safe use of various chemicals, biologicals, toxic compounds and/or other potentially harmful substances for research or other purposes. The laboratory may be equipped with one or more "special exhaust devices" that are designed to exhaust air from the lab to an outside environment to protect lab users from potentially dangerous exposure to harmful substances. For example, a laboratory may include one or more of the following special exhaust devices such as laboratory fume hoods, canopy hoods, glove boxes, or non-recirculating biological safety cabinets, in which potentially harmful substances may be regularly handled. Additionally, exhaust trunks sometimes referred to as snorkel exhausts are special exhaust devices that may be used to exhaust air containing potentially harmful substances from a particular area on a bench top or from an analytical instrument thereby providing local containment and protection. Additionally, a laboratory may include one or more exhausted storage cabinets that are special exhaust devices that are used to store potentially hazardous substances and function to contain harmful fumes or vapors that might leak from the stored substances. The described laboratory environment may be used for many purposes, such as research, teaching, manufacturing or production, quality control, pilot or scale up, or other functions. Additionally the laboratory may contain many types of areas adjacent to or beyond the research areas such as support rooms, equipment rooms, corridors, offices and other types of rooms found in a laboratory building that may also be one-pass environments with all the air from the spaces exhausted to outside the facility.

Another example of a critical environment in which a one-pass dilution ventilation system may be employed is a vivarium as mentioned earlier. A vivarium is generally'a facility used to house animals for research purposes. These animals which can include rats, mice, rabbits, larger mammals, and even aquatic life such as fish have many environmental requirements. In addition to proper temperature control and lighting, it is important to use containment and or dilution ventilation to reduce and exhaust odors, animal dander, particles, gases from the animal's metabolic functions, and potentially toxic gases from the animal holding and other rooms that are part of these facilities. In addition to protecting the animals, the reduction and elimination of these contaminants in the air is important to the health and safety of the animal care and research staff who use these facilities. In particular, the exposure of these people to animal allergens, such as rat urine protein (RUP) or mice urine protein (MUP), that is often carried in the air on particulates can over time sensitize the animal care workers and researchers, and create allergic reactions in these individuals from the animals via contaminated air in the facility. The vivarium may also have special exhaust devices, for example the animal cage racks that are often used to house rats and mice for example have cages with filter membrane tops and are also ventilated. In particular, these racks may be directly exhausted into a general exhaust duct via a constant, 2-state, or variable air volume valve or other flow control device like a damper-based system.

Additionally, some vivarium or animal facility rooms may contain biosafety cabinets that may be exhausted, snorkel exhausts or laboratory fume hoods. Vivariums may also have many different functions similar to the lab room functions mentioned above and similarly as mentioned above may have many types of rooms such as support rooms, surgery, examination rooms, cage wash area, corridors, "clean" corridors, "dirty" corridors, offices, and other rooms that are part of the animal or vivarium facility that do not house animals yet still are one-pass environments.

In view of the foregoing, conventional ventilation processes in a laboratory facility, vivarium, or potentially other similar or related critical environments where dilution ventilation is employed, generally involve supplying 100% fresh outdoor air to the environment in the form of supply air ducted directly into the space or supply air that is supplied to other nearby spaces and then passes into the environment as transfer air or as a constant source of offset air. It should be obvious to those who are experienced with building ventilation system technology that this ducted supply air is usually provided via an air handler that contains a fan to move the air, but also will usually include a method for heating, cooling, and filtering the air. Offset airflow is specifically defined as a typically fixed difference in airflow between the ducted supply into the space and the ducted exhausted airflow from the space. Depending on whether the supply is greater than or less than the total exhaust airflow from the space determines whether the environment is at a positive or negative pressure respectively with respect to an adjacent room or corridor. As an example for labs, typically the supply air is controlled to be less than the exhaust to ensure that the lab is at a negative pressure with respect to the corridor. In the context of this invention, corridor is defined as a passageway that may be adjacent to and is in communication with a plurality of rooms or critical environments.

There are three factors that may be typically used to determine the level of supply and exhaust airflow into the critical environment. The first of these factors is the space thermal load. Typically the supply air into the space is conditioned at a temperature such as 55 degrees F and used for cooling the environment. The heat sources with in the lab can be solar load, lights, people and the so-called plug loads from the heat generated by equipment and instrumentation within the lab. As these loads increase the cool supply air must be increased to maintain a given temperature set point such as 72 degrees Fahrenheit. Occasionally, the lab may be cooled using methods other than the supply air such as from a cooled ceiling or floor or from a local fan coil unit that pulls air from the room, passing this air through cooling coils (typically chilled water coils), and passes it back into the room. In these latter two cases the control of the environment's supply and exhaust airflow would be unaffected by the thermal load factor. For purposes of this invention a single pass environment would not be altered from a pressurization standpoint by adding the aforementioned fan coil and, therefore, would be viewed as being a single pass environment even though it may be connected to a locally recirculating fan coil or other device such as a ductless fume hood, glove box, or other device that locally recirculates air from said environment while having no net influence on the offset airflow to the environment.

A second factor that can determine the environment's required supply and exhaust airflow is the exhaust airflow from any special exhaust devices as described earlier as well as the associated make up air needed to match the exhausted airflow. These sources of exhaust air may be fixed sources such as from storage cabinets or biosafety cabinets, two-state (high/low) sources or air or fully variable sources such as with variable air volume laboratory fume hoods. Alternatively, the space or environment may have no special exhaust devices and thus this factor will not affect the airflows of the space.

The third and final factor affecting the environment's required supply and exhaust airflow is the airflow requirements for dilution ventilation. This requirement is typically expressed as a certain number of air changes per hour (ACH) for the space such as 6 air changes per hour of total exhaust (including special exhaust airflow) or total supply (including offset and transfer) airflow. This number of air changes per hour can then be converted into a specific airflow rate for a given volume space. For example if an environment is 20 feet long by 25 feet wide and 9 feet high, the total volume of the space is 4500 cubic feet. Thus 6 air changes an hour would mean that in 60 minutes the entire volume of 4500 cubic feet would be exchanged 6 times, or equivalently there would be one air change in 10 minutes (60 minutes/6 ACH = 10 minutes per air change). For the volume of 4500 cubic feet to be exchanged in 10 minutes would require a total room supply or exhaust flow of 450 cfm (4500 cubic feet/ 10 minutes per air change = 450 cfm). Typical industry accepted required flows for dilution ventilation range from 6 to 12 air changes per hour.

Furthermore, this amount of air changes per hour of airflow is typically a fixed level that is set irrespective of the actual quality of the room air, even though the air exhausted from the lab environment often is clean and safe. Additionally, due to simplicity and costs, some portions of the lab or vivarium environment served by the ventilation system, such as storage areas and support areas, or even offices where there may be no hoods, animals, or active research are also ventilated with one-pass air at these levels, even though the possibility of contaminants being present in these areas is less likely. Accordingly, the minimum fixed air changes requirements for 100% outside air in conventional laboratory, vivarium, or other dilution ventilation systems often results in wasted resources (i.e., fresh outdoor air) and unnecessarily excessive operating costs as well as high up front capital costs for sufficient sizing of the building's heating, ventilating and air conditioning system also referred to as the HVAC system.

The typical approach used to integrate the three factors, or requirements mentioned above, into a single flow requirement for total exhaust or supply is simply to take the highest of the three requirements. If constant volume airflow devices are used then they must be set for the highest of the peak requirements of each the three factors. If variable volume airflow control devices are used, then the environment airflow can vary based on the highest of the actual requirements such as the variation in thermal load. Traditionally, in many one-pass, critical environments, the dominant factor that has been the controlling factor has been either the thermal load or the requirements of the special exhaust devices such as laboratory fume hoods.

As such there have been many inventions and technologies developed to safely vary the environment's airflow to save energy based on either or both of varying airflow to meet the actual thermal load requirements, or varying the airflow through the special exhaust devices. The latter has often been done through the use of variable air volume laboratory fume hoods; such as those described in U.S. Patents Nos. 4,706,553; 4,893,551 and 5,240,455; since fume hoods have often been the dominant driver behind laboratory airflows. More complex airflow controls involving the sensing of air contaminants in critical environments that would typically be dominated by thermal loads have also been developed to safely vary and recirculate air from critical environments, such as described in U.S. Patents Nos. 2002/0072322; 6,609,967 and 6,790,136 through the addition of a return airflow control device to each critical environment to return and reuse clean air in an air handler serving multiple lab rooms.

In the last five to ten years, there have two important trends that have affected the airflow levels in labs. First, the numbers of laboratory fume hoods and related special exhaust devices has decreased. This is partly related to the increased use of computers to model chemical reactions vs. lab experimentation, as well as the use of smaller amounts of chemicals in research. Additionally, more life sciences labs that tend to have less fume hoods are being built today vs. the traditional chemistry lab with many fume hoods. Furthermore, many labs today are built with variable air volume laboratory fume hood control systems to reduce the amount of exhaust and make up air related to the fume hood. As a result the lab ventilation requirements related to special exhaust or fume hood make up air have been reduced significantly, so that in many labs it is not the driving force determining the airflow or ventilation in the lab.

Second, thermal loads in labs have also dropped as more energy efficient technologies are being used in labs. The efficiency and waste heat from lighting for example has dropped significantly as has the power used by lab instrumentation. Although in the early 90's the amount of lab instrumentation increased significantly, over time this equipment has become smaller and more energy efficient. Refrigerators and freezers have in many cases dropped their power consumption by two-thirds, plus LCD displays and laptops have replaced desktop computers and large energy hungry CRT monitors. Many recent studies show this result, such as a study from the Lawrence Berkeley National Laboratory mentioned in the September, 2005 issue ofHPAC Engineering entitled "Right-sizing Laboratory HVAC systems". This article demonstrates that labs are often over designed for thermal loads that are 5 to 10 times more than what the lab environment will actually be used for.

As a result of these two trends, the minimum or dilution ventilation requirement has emerged as often the dominant and controlling factor in laboratory airflow requirements. If this level can, on average, be reduced it would save significant amounts of energy in laboratories as well as allowing a smaller HVAC system that would save first cost in the construction of the facility. As a point of fact, the level of minimum air change or dilution ventilation requirements are also usually set somewhat arbitrarily, for example at levels of between 6 to 12 air changes per hour for a laboratory or 10 to 20 ACH's for a vivarium.

Occasionally to save energy, this dilution rate is made a two state flow reduced during unoccupied times to a set lower level such as 4 ACH and then increased during occupied times to a higher level such as 8 ACH. This control can occur by a set time schedule control or through the use of an occupancy sensor such as those commonly used to shut off lights. Although this approach can save energy it has several safety problems that negate its prudent use. For example, a spill or release of hazardous vapors can occur during an unoccupied time increasing the level of contaminants in the air above safe levels. If someone were to walk into the space during this scheduled unoccupied time, they could be injured by the higher level of contaminants in the air. Even with an occupancy sensor or detector, when the individual walked into the room the level of contaminants could be quite high, exposing the individual until the system both detected their presence and more importantly was able to adequately flush out the lab with the higher occupied airflow which could take some time. Furthermore, occupancy detectors can have problems with detecting people in a broken up space with many barriers such as lab shelves and equipment between the sensor and the occupants. They also need to constantly see motion to operate and may fail to see someone quietly reading with insufficient motion to trigger the higher safe airflow. If the flow is dropped due to lack of sufficient motion, the occupant might not notice the flow change and then even worse could possibly be overcome by a higher level of contaminants in the lab.

### SUMMARY OF THE INVENTION

It is therefore a primary object of this invention to provide a system for controlling ventilation to dilute contaminants within critical environments or spaces, such as laboratories and vivariums which utilize a "one-pass" ventilation strategy in which the airflow out of each environment is entirely exhausted without a recirculated air component.

It is a further object of the invention to provide a system for varying the flows of supply and exhaust air into and from these environments for the purposes of controlling the dilution of air contaminants based on changes in the presence of these contaminants as sensed by a facility monitoring system.

Accordingly, the present invention provides a dilution ventilation control system for use in a one-pass critical environment according to claim 1.

The system of the invention was developed using a novel, improved approach, from both a safety and an energy savings perspective over the prior art use of a time clock or occupancy sensor to vary minimum ventilation levels, to instead vary the amount of dilution ventilation airflow or equivalently defined in the context of this invention, the air change rate of a space, based on the level of an air contaminant in the space as measured by a facility monitoring system. In the context of this invention a facility monitoring system is defined as a monitoring system that includes at least one air contaminant sensor that measures at least one air contaminant of at least one room, space, area or critical environment. Such a facility monitoring system may involve the use of one or more individual, local, wired or wireless sensors located in the space being measured. It may also use remote or centralized air contaminant sensors that are multiplexed or shared amongst a plurality of spaces as is described in more detail later. Finally, a facility monitoring system may use a combination of the previously mentioned remote and local air contaminant sensors. As such these facility-monitoring systems may be used to measure many different air contaminants as well as potentially other characteristics of the monitored space such as temperature, humidity, or differential pressure with respect to some other space.

An air contaminant sensor in the context of this invention refers to a sensor that converts the level of or information about the presence of an air contaminant into either a continuously varying or else discontinuous pneumatic, electronic, analog or digital signal or else into a software or firmware variable representing the level of or information about the presence of an air contaminant in a given space. The air contaminant sensor may be based on any of a variety of sensing technologies known to those skilled in the art such as for example electrochemical, photonic or optical, infrared absorption, photo-acoustic, polymer, variable conductivity, flame ionization, photo-ionization, solid state, mixed metal oxide, ion mobility, surface acoustic wave, or fiber optic. The air contaminant sensor may be a wired or wireless sensor type and be implemented with various types of physical hardware such as for example micro-electro-mechanical system based (MEMS), nanotechnology based, micro-system based, analog based, or digital based. Additionally, an air contaminant sensor may sense for more than one air contaminant, may include more than one air contaminant sensor in one packaged device, or may sense for or include sensors for other non-contaminant air parameters such as for example temperature, pressure, or a measure of humidity. In the context of this invention, air contaminants refers to certain chemical, biological, or radiological composition elements or properties of the air such as for example carbon monoxide (CO), particles of various sizes, smoke, aerosols, TVOC's (Total Volatile Organic Compounds), specific VOC's of interest, formaldehyde, NO, NOX, SOX, SO2, nitrous oxide, methane, hydrocarbons, ammonia, refrigerant gases, radon, ozone, radiation, biological and or chemical terrorist agents, mold, other biologicals, and other chemical characteristics of the air and contaminants of interest to be sensed. Also, in the context of this invention the term air contaminants specifically does not include or refer to such air characteristics or parameters such as any measure of temperature, carbon dioxide, or humidity such as for example any of the linked measures of temperature and moisture or water vapor in the such as relative humidity, absolute humidity, wet bulb temperature, dry bulb temperature, dew point temperature, or grains of moisture per pound of air. Additionally, in the context of this invention, air contaminants also does not specifically include or refer to any measure of airflow volume, velocity or pressure such as air volume as may be indicated in units of cubic feet per minute of air or other units, velocity pressure, air speed or velocity, static pressure, differential pressure, or absolute pressure.

The air in modern laboratories is often quite clean such that high air change rates are unnecessary except for example when a spill happens; poor lab practices generate fumes, vapors or contaminants in the lab outside the containment devices like fume hoods; or when the containment devices work poorly leaking chemical fumes into the space. Since the far majority of the time the lab air is clean, the dilution ventilation airflow can be brought down significantly the majority of the time to a level such as 2 to 4 ACH's vs 6 to 12 ACH's creating significant ventilation savings. Additionally when a spill occurs, the system can increase the dilution ventilation rate to a high level such as 12 to 15 ACH, providing increased safety through a fast evacuation of the spilled vapors from the lab.

Dynamic control of dilution ventilation based on monitoring the quality of air with a facility monitoring system can be accomplished with several different embodiments. Perhaps the simplest approach to dynamically vary air change requirements in one-pass, critical environments, such as labs or vivariums, would be to use a single, broad based contaminant sensor such as a TVOC or total volatile organic compound sensor located in each room or airflow control zone that is to be controlled. This approach can for example increase the dilution ventilation airflow requirements when the contaminant sensor detects that contaminants are above a given threshold level. When the contaminant level returns below the given threshold level, the dilution ventilation airflow requirement is brought back down to the minimum set point level. In all conditions if the thermal load or special exhaust make-up airflow requirements are above the required dilution ventilation flow requirements, then these requirements will override and take control of the room's airflow level in a high select form of control.

There are several important issues to be taken into account when implementing this type of one sensor per room/airflow control zone approach to the dynamic control of dilution ventilation. First of all, the selection and use of lower cost, typically metal oxide type TVOC sensors may create problems due to the relatively high drift and even poisoning and degradation that can often occur with these types of sensors when they are exposed to certain airborne contaminants that are likely to be found in labs, vivariums or other environments where outside air is used to dilute airborne contaminants. A preferred alternative would be to use a higher-grade TVOC sensor such as for example a photo-ionization detector (PID) style sensor. These sensors although typically more expensive, are also more stable and much less apt to be compromised by the gases they are detecting.

Additionally, the use of a TVOC sensor, even a PID type TVOC sensor, will not detect all contaminants of concern in a lab. For example, there could be a release of an aerosol, hazardous particles, or smoke from an out of control reaction that needs to be rapidly evacuated from a lab. Similarly, non-organic acid gases, for example, could be quite harmful to a researcher but similarly will not be detected by a TVOC sensor. Furthermore, there may be certain specific contaminants that are of concern that would be beneficial to sense such as formaldehyde or ammonia. Finally, depending on the allowed minimum air change level, if a space may be heavily occupied, such as with a teaching lab, CO2 monitoring may be needed as a means of detecting heavy occupancy levels and increasing airflow to meet outside air requirements and guidelines related solely to the amount of people in the space such as to meet a typically used guideline of 15 to 20 cfm of outside air per person. As such an embodiment that can be used to deal with these issues would use multiple sensors vs. just one sensor in one or more rooms or where it is appropriate to detect some of these other contaminants to provide safe operation of the system and potentially also to sense CO2 for additional airflow control related to occupancy.

An exemplary embodiment of the current invention that provides another solution to these issues and is both practical and cost effective is the use of a multipoint air sampling system, otherwise known as a multiplexed or shared sensor based facility monitoring system, as the means to sense the quality and cleanliness of the lab environment. Multipoint air sampling system are defined for the purposes of this patent as specifically a facility monitoring system that uses shared or multiplexed sensor(s) consisting of either a single remote sensor or a set of remotely located sensors that is used to monitor a plurality of spaces, areas or rooms within a building, or outside adjacent to a facility by transporting samples or packets of air from the critical environment to be monitored to the at least one air contaminant sensor.

For one class of these multipoint air sampling systems specifically defined, in the context of this invention, as star configured multipoint air sampling systems or just star configured systems, multiple tubes may be used to bring air samples from multiple locations to a centralized sensor(s). Centrally located air switches and/or solenoid valves may be used in this approach to sequentially switch the air from these locations through the different tubes to the sensor to measure the air from the multiple remote locations. Each location may be sensed for between 10 seconds or several minutes. Depending on how many locations are sensed each space may be sensed on a periodic basis that could range from 5 to 60 minutes. These star configured systems may be called octopus-like systems or home run systems and may use considerable amounts of tubing. An example of such a star configured system is described in U.S. Patent No. 6,241,950, which is incorporated herein by reference. Other types of known air monitoring systems include those that have been designed to monitor refrigerants and other toxic gases, which also are star configured systems. Additionally, these types of star configured systems have been used to monitor particulates in multiple areas such as clean room areas with a single particle counter. Generally, these types of systems have not historically been applied to general air quality measurement applications involving multiple parameters such as TVOC's.

Another multipoint air sampling system defined in the context of this invention as a networked air sampling system uses a central "backbone" tube with branches extending to various locations forming a bus-configured or tree like approach similar to the configuration of a data network. Air solenoids are typically remotely located proximate to the multiple sampling locations. The sampling time for each location like with the star configured systems may vary from about 10 seconds to as much as several minutes. A typical sampling time per location would be about 30 seconds, so that with 30 locations sampled, each location could be sampled every 15 minutes. Networked air sampling systems can also include remote and/or multiple-location air sampling through a tube or pipe for sampling locations in a building, outdoor air or ambient sampling, and exhaust air stacks. An exemplary networked air sampling system is described in U.S. Patent No. 6,125,710.

Finally another multiplexed form of facility monitoring system that may be used to implement portions of this invention is defined in the context of this invention as a networked photonic sampling system that multiplexes packets of light vs. packets of air and may incorporate either a star configured or network/bus type of layout. The basic concept uses a central laser emitter and a central laser detector that sends out and detects laser light packets that are switched into rooms to be sensed by optical switches. Optical fiber sensors, infrared absorption cells or sensors, and other sensing techniques are located and used in the sensed area to change the properties of the light due to the affect of the environment. The light packet is then switched back to the central detector where the effect of the environment on the light properties is determined. A major benefit of the system is that the sensors such as the fiber or open cell sensors are potentially quite low in cost. The expensive part is the laser and detector systems that are centralized. Like in the previous multipoint air sampling systems, multiple affects on the light from particles, gases and other contaminants, humidity, etc. can be done simultaneously with central equipment and the telecom concept of Wavelength Division Multiplexing which allows multiple wavelengths and hence multiple signals to share the same fiber. A clear advantage of this system is the ability to have a cycle time that can be in ten's of milliseconds or less. This sampling system is detailed in U.S. Patent No. 6,252,689, entitled "Networked Photonic Distribution System for Sensing Ambient Conditions".

The multipoint air sampling systems and networked photonic sampling system which have been described heretofore and are collectively referred to as sampling systems may be applied to monitor a wide range of locations throughout a building, including any kinds of rooms, hallways, lobbies, interstitial spaces, penthouses, outdoor locations, and any number of locations within ductwork, plenums, and air handlers. To provide control as well as monitoring of these different spaces, virtual sensor signals can be created that in the context of this invention refer to software or firmware variables, or continuous analog or digital signals that can be passed to other systems such as a building control or laboratory airflow control system and are representative of the state of a given space's air contaminant value. In effect these signals are reflective of what a local sensor would read if it was being used instead of the multipoint air sampling system or networked photonic sampling system otherwise known collectively again as sampling systems.

Another characteristic of these sampling systems is that some characteristics or parameters of the air such as temperature in particular, as well as some air contaminants such as potentially ozone can not always be effectively measured from a remote location with a shared sensor. Furthermore, other contaminants may be accurately measured at a remote location with a shared sensor but, for various reasons such as the need for more rapid sampling, may be preferably sensed locally at one or more of the sensed locations. In these situations, separate local sensors and either distinct signal wires or a digital data communications network with cable, optical fiber or wireless links can be used to connect these local sensors such as temperature sensors to either the networked air sampling system, star configured multipoint air sampling system, networked photonic sampling system, or possibly a building management system. These virtual sensor signals plus potentially local sensor signals can be combined to create blended signals that may be used advantageously for monitoring and or control purposes as described in U.S. Patent Application entitled, "MULTIPOINT AIR SAMPLING SYSTEM HAVING COMMON SENSORS TO PROVIDED BLENDED AIR QUALITY PARAMETER INFORMATION FOR MONITORING AND BUILDING CONTROL" and filed on March 10, 2006.

When the multipoint air sampling systems are used to sample ductwork, plenums, air handlers or any other applications where flowing air in a partially contained area such as a duct or pipe is to be sampled and measured with a remote sensor, a tube or hollow duct probe may be inserted into the duct or partially contained space to withdraw a sample or else a hole can be made in the duct and a sample drawn from the duct from a tube connected to the opening in the duct wall. Additionally however, a separate temperature or other parameter or contaminant sensing probe or probes are needed to make whatever local sensor measurements are desired from these ducts or partially enclosed areas. Multiple separate probes for both sensing the flowing air stream and for drawing air samples may be employed or a unique integrated sampling probe that uses one probe for both local air characteristic measurements and for air sampling may be used as described in the U. S. Patent Application Serial No. 11/312,164, entitled "DUCT PROBE ASSEMBLY SYSTEM FOR MULTIPOINT AIR SAMPLING".

Another embodiment of the current invention uses the virtual signals from a multipoint air sampling system and or the signals from local room or duct air contaminant sensors and combines them via one or more of multiple approaches using a signal processing controller to create a dilution ventilation command signal that in the context of this invention is an airflow command signal that can be used to vary at least partially the dilution ventilation airflow or air change rate of a critical environment based on one or more air contaminants.

For the purposes of this patent, an airflow command signal is any pneumatic, electronic, analog or digital signal, or a software of firmware variable that operates in a firmware or software program running on a microprocessor or computer, that is used by either the critical environment airflow controller or by one of the room exhaust, special exhaust or supply airflow control devices to at least partially vary or control one of the aspects of or relationships between any one of the airflows moving into or exiting the critical environment. This airflow command signal may be of a continuously varying nature and is otherwise referred to herein as a VAV or variable air volume command signal. Otherwise, the airflow command signal may be a discontinuous airflow command signal which in the context of this invention is defined as a signal that may have only two levels or states and for the purposes of this patent is referred to as a two state signal, or it may have three levels or states and may thus be referred to in the context of this invention as a three state signal. Alternatively, the discontinuous airflow command signal may have multiple discrete levels or states and as thus may be referred to herein as a multiple state signal.

When multiple air contaminants are to be used by a signal-processing controller to create a dilution ventilation airflow command signal, particularly where each contaminant has a different threshold of concern, each contaminant can be scaled to a standard scale relative to that threshold. For example 2 volts in a 0 to 10 volt scale can represent the threshold at which point the airflow begins to be increased with 10 volts representing maximum flow. These individual signals can then be high selected so the higher of these signals controls the dilution flow. Alternatively, the signals can be summed together after they have been weighted in a relative manner based on the severity of the health effects of each sensed compound.

Another problem of concern with the dynamic control of dilution ventilation based on the measurement of air contaminants in a space is that if outdoor levels of a contaminant go high due to re-entrainment of "dirty" exhaust flows, high outdoor dust levels, traffic, etc, the system could be triggered by these levels and increase the supply airflow into the rooms. This action would actually make matters worse and would "latch" virtually all the controlled spaces up at the high ventilation level. Since the system capacity would have been likely not designed for each space operating at maximum flow then, this event would call for a system capacity that could not be achieved. This potentially could compromise the airflow control throughout the building, reducing flows, and thereby creating loss of capture with the special exhaust devices as well as potentially compromising room pressurization levels. A similar problem of exceeding system capacity could also be achieved if for example the hallways of a lab or vivarium are being mopped with a cleaner that gives off VOC's. The floor cleaner fumes could be quickly pulled into many of the negatively pressurized lab rooms thereby triggering many of the lab spaces into a high flow level state, thereby creating a similar capacity problem.

To prevent multiple spaces from going to high dilution ventilation incorrectly due to a high outdoor level of contaminants, another embodiment of the current invention describes a means to vary the dilution ventilation of a space not on the absolute value of a given contaminant, but instead on the differential value of that air contaminant vs. either an outdoor air value, a supply airflow value, or the value measured in an adjacent or nearby space. In this manner the room does not incorrectly increase the flow of a contaminated supply air stream, when the contaminant sensed is not from inside the room. In a related embodiment, if the absolute level of the room exceeds the threshold value for action, yet the source of the contaminant is from the supply of outside air, the supply air may be decreased. For example, the supply air may be decreased by commanding a lower dilution ventilation level and/or commanding an increased temperature set-point, to reduce the thermal load requirements on the supply volume.

For those critical environments where there is a concern that a spill or contamination of the room could spread to other rooms or for other reasons requiring increased containment or protection, a further embodiment of the present invention could increase the exhaust air and decrease the supply air of the affected room or area to increase the negative pressure offset of the contaminated room to increase the level of containment of that space vs. other spaces. If a given room is believed to the source of a potential contamination, then surrounding rooms could be increased to a positive pressure to further isolate the contaminant.

For the purposes of this patent a signal processing controller as mentioned above refers to analog or digital electronic circuitry, and or a microprocessor or computer running a software or firmware program that uses at least information, signals and or software or firmware variables from either individual local sensors of air contaminants and or other air characteristics such as temperature, humidity, air volumes, or pressures plus virtual sensor signals, information and or software or firmware variables from remote, centralized sensors of air contaminants, and combines and processes this information in a potential multitude of ways. As a result the signal processing controller either creates airflow command signals for dilution ventilation, offset air volumes, or other airflow commands to be used by a critical environment airflow controller, and or create signals or information that can be used by other control devices such as a building control system for at least partially controlling one or more critical environment airflows of supply, room exhaust, special exhaust or offset airflow, and or is used for some other control or monitoring function that is in some way related to the control of one of the aforementioned critical environment airflows.

In the context of this invention, a building control system or building management system as mentioned above is defined as a control system located in a building or facility that is used to control one or more functions of the HVAC system in a building such as for example control of space temperature, space relative humidity, air handling unit airflows and operation, exhaust fan flows, chiller operation, duct static pressures, building pressurization, critical environment airflows. These systems often integrate with or incorporate other building systems or subsystems such as fire and security, card access, closed circuit TV monitoring, smoke control systems, power monitoring, and critical environment airflow control systems. Building control systems may have pneumatic, electric, electronic, microprocessor, computer, or web based controls using pneumatic, analog and or digital signal inputs and outputs. These systems often have centralized monitoring functions, centralized or local control capabilities, and may have Internet or web based access. They may also be referred to as building management systems (BMS), facility control systems or facility management systems (FMS).

Finally, there may be improved or at least alternative approaches to increasing ventilation in a lab that has a spill event. For example is the door of the space to the corridor has been left open, the slight negative pressure of the lab room vs. the corridor may not be enough to prevent the spill vapors from contaminating other adjacent areas. It may be better to specifically have the system react in some other way to ensure that the vapors are contained in the room with the spill.

### BRIEF DESCRIPTION OF THE DRAWINGS

Other objects, features and advantages will occur to those skilled in the art from the following description of the preferred embodiments and the accompanying drawings in which:
FIG. 1 is a schematic diagram of a preferred embodiment of the system of the invention in which a plurality of one-pass, critical environments are being monitored by a multipoint star configured air sampling system.
FIG. 2 is a schematic diagram of a preferred embodiment of the system of the invention in which a plurality of one-pass, critical environments are being monitored by a multipoint networked air sampling system.
FIG. 3 is a detailed schematic diagram of a preferred embodiment of the system of the invention in a one-pass, critical environment.
FIG. 4 is a schematic diagram of a portion of a preferred embodiment of the signal processing logic of the invention that may be used to create the dilution ventilation command signals.
FIG. 5 is a schematic diagram of an embodiment of the critical environment airflow controls logic of the invention for a one-pass, critical environment space including one or more special exhaust sources.
FIG. 6 is a schematic diagram of an embodiment of the system of the invention in which a plurality of one-pass, critical environments are being monitored by one or more of individual local sensors comprising one or more unique features.
FIG. 7A and 7B are schematic diagrams of various steady-state levels associated with air change rate control sequences.
FIGS. 8A and 8B are diagrammed strategies for controlling the air change rate in critical environments using a closed loop system to provide dilution ventilation control by varying the air change rate within a critical environment.

### DETAILED DESCRIPTION OF THE PREFERRED EMBODIMENTS AND METHODS

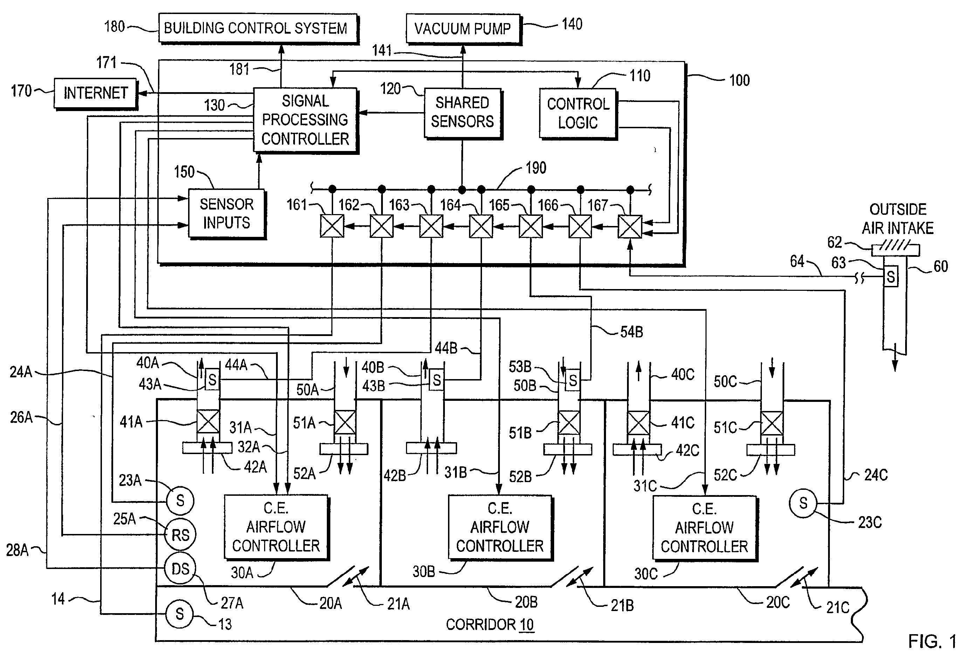

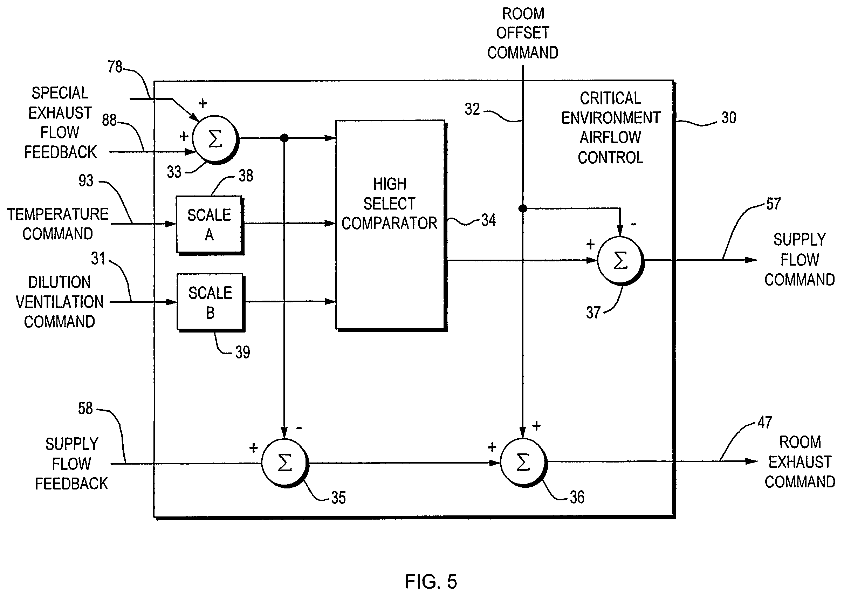

FIG. 1,2 and 6 all show a typical set of monitored critical environments or rooms 20A, 20B, and 20C that have doors entering a corridor 10 that is also being monitored. Due to a positive or negative pressurization of the critical environments, an offset may exist between the critical environment space or area and an adjacent space. An example of this is shown in FIG. 1, 2 and 6 as offset airflow 21A, 21B, and 21C between the spaces 20A, B, and C and corridor 10. Although the diagrams show three rooms and a corridor, the present invention may be used with any plurality of rooms or spaces including corridors or other adjacent spaces that are also being monitored, such as for example, two or more critical environments, or one corridor plus one or more spaces. Note also that, although the critical environments shown in the Figures are enclosed within walls, critical environments in the context of this invention may also be a section or area of a room having no walls or partitions around it. Thus, there may be multiple critical environments within one physical room. Alternatively, multiple physical rooms may also constitute one critical environment or space. Typically, the critical environment 20 will also be an area that is fed by one or more supply airflow control devices 51 plus one or more room exhaust devices 41 that are being controlled by a critical environment airflow controller 30 as one airflow control zone. For the purposes of this patent a critical environment airflow controller is an airflow control apparatus that may be of analog or digital electronic design or may be constructed using a microprocessor or computer running a software or firmware program that creates the airflow command signals for one or more supply and or exhaust airflow control devices possibly using information, signals and airflow commands from other devices, systems or controllers. FIG. 5 shows one embodiment of a critical environment airflow controller.

These sets of rooms in FIG. 1,2 and 6 are further described as having a source of supply air from supply air ducts 50A, 50B, and 50C that may exit the room as room or general exhaust air from room or general exhaust ducts 40 A, 40B, and 40C. Although not shown in the figures, the corridor 10 often has a source of supply air and possibly room exhaust as well. The supply ducts 50A, B and C also contain airflow control devices 51A, B, and C. which supply air into the room or space through supply flow grill or diffuser 52A, B, and C respectively. Additionally, the room exhaust ducts 40A, B, and C contain room exhaust airflow control devices 41A, B, and C which control the amount of room or space air pulled into the room exhaust duct through a room exhaust grill or vent opening 42A, B, and C respectively.

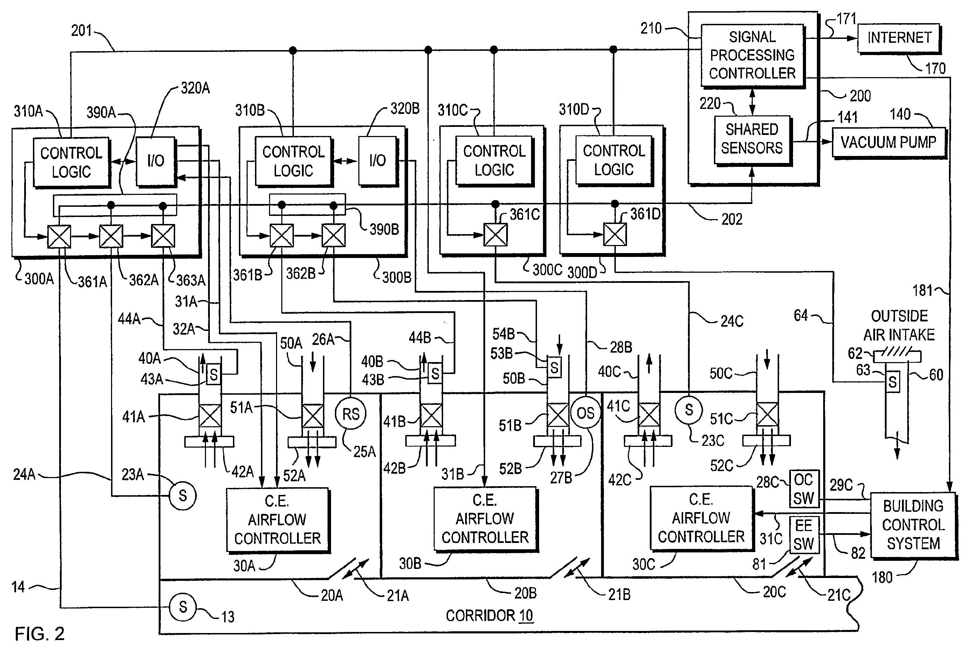

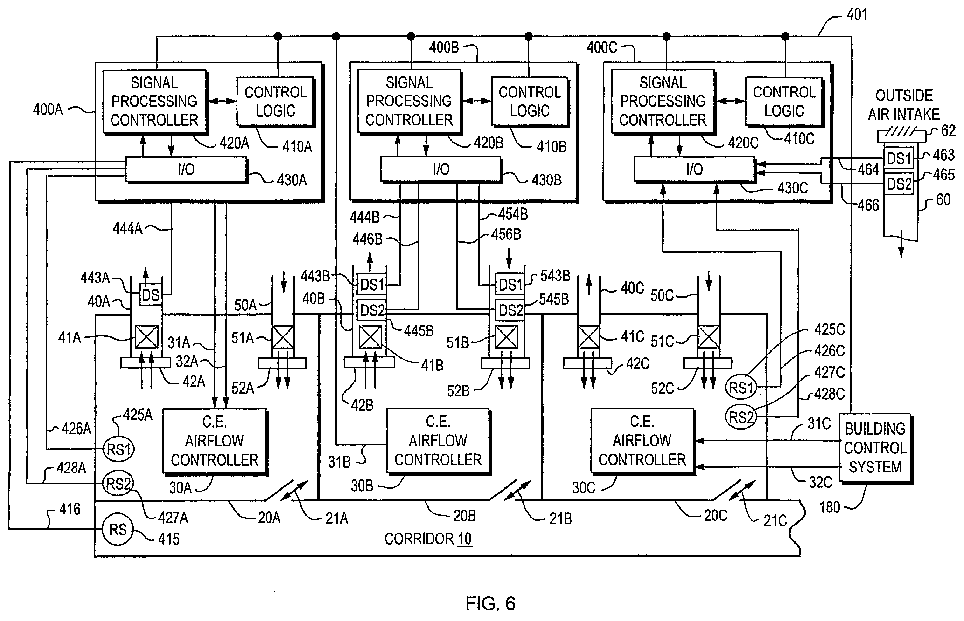

FIG. 1,2, and 6 also show the presence of an outdoor air intake 62 into the building through outside air duct 60. This duct could be connected to or part of some type of an air handling unit to pull in outside air into the building or may be a special duct or outside air pickup location specifically used for or shared by the air sampling systems 100 and 200 of FIG. 1 and 2 respectively or the air contaminant sensing system of FIG. 6.

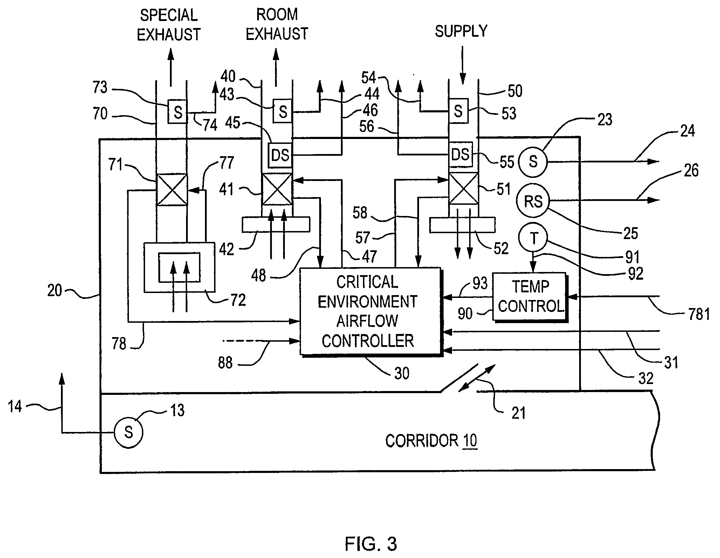

An airflow control device as used in the context of this invention, such as the supply and room exhaust airflow control devices 51 and 41 respectively or the special exhaust airflow control device 71 shown in FIG. 3, is defined as any device known to those skilled in the art of airflow control for controlling air flow volume and velocity through a duct. For example, they can be constant volume, two state, multiple state, or variable air volume (VAV) boxes or terminals such as manufactured by Titus, Metal Aire, Enviro-Tec, or others. These devices use a damper or throttling device of some type such as a single round, square, or rectangular blade damper, a multiple blade damper, a set of pneumatic bladders that can be used to seal off an opening, or any other type of throttling device that can be used to seal off a duct, that is connected to a pneumatic, electric, or electronic actuator that is controlled by a pneumatic, electronic, digital, or microprocessor based controller which typically also relies on feedback of flow from a flow sensor for closed loop control of the duct's air volume. These flow sensors can be of various types known to those skilled in the art, such as those based on single or multiple velocity pressure sensors, hot wire, heated thermistor, microelectronic flow sensor, etc. Alternatively, another type of flow control device that is commonly used is an airflow control valve that typically has a venturi shaped body with a spring loaded cone that moves through the venturi shaped throat of the device to provide inherent, pressure independent control of volume, such as manufactured by Phoenix Controls or others. These valves typically have pneumatic, electric, or electronic actuation to provide constant volume, two-state, multiple state, or variable air volume control. These devices often have large turndown or flow ranges that make them very appropriate for dynamic control of dilution ventilation that can have wide flow ranges to achieve optimum energy savings and safety. Finally, another example of an airflow control device may be some form of a single or multiple blade damper or other type of throttling device that is located either in an air handling unit or a duct serving one or more areas or potentially multiple critical environments which further includes one of the airflow measuring devices aforementioned or similar airflow measuring devices that are adapted using a grid of sensors or sensing holes for example to measure the airflow accurately across a large cross sectional duct area.

Although not shown in FIG. 1,2, and 6 the critical environments 20A, B, and C may also have one or more special exhaust airflow devices as mentioned above which also pull air out of the critical environments. An example of this is shown in FIG. 3 as special exhaust device 72, and special exhaust airflow control device 71 which will be both explained in more detail later.

With reference to FIG. 1, this diagram refers to a preferred embodiment of the present invention directed to dynamic control of dilution ventilation in one-pass, critical environments using a star configured multipoint air sampling system 100. Multipoint air sampling system 100 could be a star configured multipoint air sampling system with a structure like that described in U.S. Patent No. 6,241,950; U.S. Patent No. 5,292,280; U.S. Patent No 5,267,897; U.S. Patent No. 5,293,771 or U.S. Patent No. 5,246,668. It could also be a refrigerant and toxic gas monitor adapted for this purpose such as the Vulcain Inc. multipoint sample draw gas monitor model number VASQN8X as can be seen on their website at www.vulcaininc.com or a multiplexed particle counter such as the Universal Manifold System and Controller made by Lighthouse Worldwide Solutions, Inc., as can be seen at their website at www.golighthouse.com, coupled with one of their particle counters such as their model number Solair 3100 portable laser based particle counter or an obscuration based particle sensor. It could also be a star configured multipoint air sampling system like that of the AIRxpert 7000 Multi-sensor, Multipoint Monitoring system manufactured by AIRxpert Systems of Lexington, Massachusetts, as can be seen at their website at www.airexpert.com.

In FIG. 1, a set of solenoid valves 161 through 167 is part of a multipoint air sampling system 100. Equivalently, these solenoids 161 through 167 could be replaced with other switching means such as SSS-48C Single Scanivalve System manufactured by the Scanivalve Corporation of Liberty Lake, Washington as can be seen on their website, www.scanivalve.com, which uses a pneumatic selector switch and stepper motor to connect one of many input ports to an outlet port which can be connected to a sensor such as a pressure sensor. The solenoid valves 161 through 167 are controlled to switch in a sequence by control logic 110. This sequence may be a simple sequential pattern of one solenoid after another, or varied for example through programming to be one of potentially many preset patterns, or it can have a pattern that can be interrupted and changed to a new sequence by manual or remote command or by a trigger event based on the values or signal pattern of one or multiple sensed contaminants. This trigger event could be generated from outside the multipoint air sampling system 100 or could be created from the sensor information processed by signal processing controller block 130.

The solenoid valves 161 through 167 are connected to sampling locations 13, 23A, and 23C in the spaces as well as duct sensing locations 43A, 43B, 53B, and 63 through tubing 14, 24A, 44A, 44B, 54B, 24C, and 64. In FIG. 1 for example, sampling location 13 in corridor 10 is connected through tubing 14 to solenoid 161. Area sensing locations 23A and C in rooms 20A and C are connected through tubing 24A and C to solenoids 162 and 166 respectively. Room exhaust duct sampling locations 43A and B are connected through tubing 44A and B to solenoids 163 and 164 respectively. Supply duct sampling location 53B is connected through tubing 54B to solenoid 165. Finally outside air duct sampling location 63 is connected through tubing 64 to solenoid 167. Alternatively, tubing 64 may be connected to some other suitable location other than duct 60 to obtain outside air samples.

The tubing mentioned above transports the air sample from the sensing location to the solenoid of the multipoint air sampling system 100. The tubing typically will have an inner diameter of one eighth to one half an inch in diameter with a preferred inner diameter of about one quarter inches. This tubing can be made of standard plastic pneumatic tubing such as Dekoron TM low density polyethylene (LDPE) plastic, Teflon, stainless steel, "Bev-A-Line XX " tubing made by Thermoplastic Processes, Inc. of Stirling, NJ, or other suitable tubing materials known to those skilled in the art. For superior performance in transporting both TVOC's and particles however, a material that is both inert to VOC's with very little adsorption and desorption as well as electrically conductive to prevent static buildup is preferred such as flexible stainless steel tubing. Other preferred materials and constructions are described in U.S. Patent Application Serial No. 10/948,767, filed on September 23, 2004 entitled, "TUBING FOR TRANSPORTING AIR SAMPLES IN AN AIR MONITORING SYSTEM", as well as U.S. Patent Application Serial No. 11/149,941 filed on June 10, 2005, entitled, "AIR MONITORING SYSTEM HAVING TUBING WITH AN ELECTRICALLY CONDUCTIVE INNER SURFACE FOR TRANSPORTING AIR SAMPLES".

Additionally in FIG. 1, a vacuum pump 140 pulls air from the sensing locations through the tubing into the solenoids 161 through 167 and into a manifold 190 connecting all the output ports of the solenoids together and to the inlet of the shared sensors 120. The outlet of the shared sensors 120 is connected to the vacuum pump by tubing 141, whose construction is not critical and can be inexpensive plastic tubing such as the Dekoron TM mentioned above or other. The inner diameter of this tubing can be made similar to the size of the tubing connecting to the inlets of the solenoid valves or possibly larger for less pressure drop. The shared sensors 120 can consist of one or more sensors to measure such air characteristics as humidity, CO2, dewpoint temperature, and differential static pressure., as well as air contaminants such as for example, CO, particles, smoke, TVOC's, specific VOC's of interest, formaldehyde, NO, NOX, SOX, nitrous oxide, ammonia, refrigerant gases, radon, ozone, biological and or chemical terrorist agents, mold, other biologicals, and other air contaminants of interest to be sensed. These sensors may be connected in series, in parallel or a combination of both.

The signal outputs of the shared sensors 120 are passed to the signal processing controller block 130 of the multipoint air sampling system 100. This block 130 also accepts other sensor information from the sensor inputs block 150. This input block 150 accepts sensor signals or information from local room or duct sensors if needed or desired rather than remote sensors. For example, temperature cannot be sensed remotely, since the temperature of the air will change as it moves through the tubing. Additionally, some areas may need instantaneous sensing or the input may not be a sensed contaminant of the air such as the state of a room switch or an occupancy sensor. This is shown in Room 20A where room sensor 25A, which could for example be a temperature sensor, is connected to the sensor inputs block 150 through electrical cable 26A. Additionally optional occupancy sensor 27A, which may provide a digital high/low signal indicating the presence of someone in the space, is connected to sensor inputs block 150 through cable 28A. The sensors and the sensor inputs block may accept many signal forms such as analog or digital. Alternatively, the sensor may have its own onboard microprocessor and communicate with the sensor inputs block 150 through a data communications protocol such as, for example, LonTalk by Echelon Corporation, or an appropriate protocol outlined by ASHRAE's BACnet communications standards, or virtually any other appropriate protocol, including various proprietary protocols and other industry standard protocols commonly used to provide data communications between devices within a building environment. Typically, however, when digital data communications are used to connect to discrete devices such as 25A, and 27A, this is accomplished using a protocol operating over a physical layer such as an EIA485 physical layer, on top of which a suitable upper level protocol will be used. In such cases, for example, cable 28A may be specified as a twisted shielded conductor pair. Nevertheless the connections between sensors 25A and 27A and inputs block 150, may be accomplished using any number of cable types common to the building controls industry. Additionally, cable 28A may be omitted and the sensors 25A and 27A may communicate wirelessly to inputs block 150.

The signal processing controller block 130 is used to process the sensor information from the shared sensors to create virtual sensor signals reflective of the environmental conditions in the sensed locations. This information is added to the information from any local room sensors such as 25A and 27A, and is then used in a variety of possible ways. For example, this information can be sent to building control system 180 for monitoring and or control purposes through a digital networked connection 181. The information interchange could be done using for example, a BACnet protocol, Lonworks, XML data interchange or other suitable interface information conversion. The physical connection 181 could be an Ethernet connection, EIA485 (also known as RS485) connection or other type of digital data communications connection. Another use of the data can be to send it through an internal and or external local area or wide area network for monitoring at a remote location. Additionally, the data can pass directly, or through a local area network, phone network or other suitable connecting means 171 to connect to the Internet or a dedicated network from which a website or other suitable means can be used to remotely access, display, and analyze the data from the multipoint air sampling system 100.

Most importantly, signal processing controller block 130 can also provide the control signals 31 and 32 used by the critical environment airflow controller 30 which in FIG. 1 is shown as block 30A, B, and C and dilution ventilation command signals 31A, B, and C plus room offset command signal 32A. Control signal 31 is used to dynamically vary the minimum air change rate or dilution ventilation level of critical environment 20A, 20B, and 20C. The control signal 32 is used to individually, or in combination, vary the offset airflow 21A, B, and C of critical environments 20A, B, and C both in magnitude and polarity or direction (positive to the corridor or negative). Also, given the flexible nature of the electronics associated with critical environment controller 30, part or all of the functions performed by signal processing controller 130 may be performed within controller 30, which can be a programmable device. In this case, signals 31 and 32 may at least in part be created within controller 30.

Referring to dilution ventilation command signals 31, the signal processing controller block 130 can produce these signals, or portions or all of the control functions can be produced by the building control system 180, as is shown for example in FIG. 2, using sensor information from the shared sensors 220 in FIG. 2 and or the local room sensors 25A and occupancy sensors 27A. Further, it should be clear that signal processing controller 130 of FIG. 1, signal processing controller 210 of FIG. 2, or signal processing controller 420 of FIG.6 need not be physically packaged within blocks 100,200, or 400 respectively and that it's possible to implement signal processing controllers 130, 210 or 420 as either standalone modules, or to integrate them with some other portion within Figures 1,2 or 6, such as, for example, room sensor 25A. There are several different control approaches for signal 31 that can be implemented by the signal processing controller block 130 of FIG. 1 as well as by the signal processing controller blocks 210 or 420 as shown in FIG. 2 or 6 respectively, or by the building control system 180. These control approaches have two important components. One component refers to the type of control approach such as two state, three or multiple states, continuously variable control, or methods involving a combination of both discontinuous and continuous control functions. The other refers to how multiple sensor signals are combined to generate a control signal. Note however, that multiple signals are not required to dynamically vary the dilution ventilation. One signal may be used alone, such as a photo-ionization detector (PID) TVOC sensor that picks up a broad range of chemical compounds, to generate the control signal. Many types of PID TVOC sensors exist and are known to those skilled in the art of TVOC sensing. Examples of one type of a PID TVOC sensor are the RAEGuard PID, the ppbRAE Plus or the MiniRAE 2000 all manufactured by Rae Systems of San Jose, CA.

One embodiment of the control approach for dilution ventilation command signal 31 is a two state control approach whereby ventilation signal 31 is maintained at it's minimum level, for example at a dilution ventilation value corresponding to, for example, 2 or 4 ACH (or some other appropriate lower value depending on what's suitable for the environment being monitored), unless a trigger event occurs that could consist of a threshold or trigger value being exceeded by the sensor signal. As mentioned before if the sensor signal consists of just one contaminant, a simple threshold or trigger value (corresponding to the value of the sensed contaminant at which some action is to be taken) can be defined. Alternatively, the trigger could consist of the signal matching in some way a specified signal pattern such as a rapid increase in level even though a specified threshold level was not achieved. The trigger event could also consist of a combination of one or more sets of threshold values and signal pattern pairs, any one of which could constitute a trigger event.

If multiple sensor contaminants are being employed such as from the shared sensors 120 and or a local room sensors 25A, the trigger event could be defined as any one of the employed sensor signals exceeding a threshold value, matching a signal pattern, or meeting the conditions of one of potentially multiple sets of threshold level and signal pattern pairs. Each sensor signal would most likely have a different threshold value level and or signal pattern that corresponds to an appropriate value for the sensed contaminant based on accepted levels of that signal related to one or a combination of health, comfort or other criteria of importance for that sensed contaminant. For example, a PID TVOC sensor would likely have a threshold level of about 0.5 to 2 PPM. A level in this range senses many materials below their OSHA TLV (Threshold Limit Value) while still not generating many false alarms by staying above normal levels of less harmful materials such as alcohol vapors. If a particle counter measuring in the range of 0.3 to 2.5 microns is used a level can be set that would not normally be exceeded such as in the range of 1.0 to 5 million particles per cubic feet, yet still pick up the evolution of smoke or some type of aerosol release into the lab room. The specific level could be set based on the level of filtration to the space, i.e. the more the filtration, the lower the level that could be used. Other sensors such as a carbon monoxide, ammonia, nitrous oxide, ozone, or other toxic gas sensor can be set directly for the TLV of the compound or for a lower level that would not normally be reached in typical operation.

Alternatively, a triggering condition could consist of a combination of two or more sensed air contaminants each reaching or exceeding a given level for that compound or meeting some signal pattern condition. For example, individually, a moderate level of fine particles such as 1.5 million particles per cubic feet, a moderate level of TVOC's such as 0.5 PPM, or a moderate level of temperature excursion to above 85 degrees might in themselves not trigger a need for increased dilution ventilation. However, the combination of all three contaminants meeting the preceding conditions could indicate a small lab fire or explosion that would definitely require an increased level of dilution ventilation.

A further implementation of a trigger condition involving multiple sensed contaminants could be an additive trigger condition. A good example of this relates to exposure to hazardous materials. OSHA indicates that the effective TLV of a mixture of gases can be computed by adding the fractions of each individual compound's level vs. it's TLV to get the fraction of the combined mixture against the combined TLV. For example, if the system detects that carbon monoxide is at 65% of the threshold limit value and that sulfur dioxide is sensed to be at 70% of its TLV value then although individually neither compound would trigger the system the combination of the two would be at 135% of the combined TLV and as such would constitute a trigger condition. To implement this approach each sensed contaminant of interest would be individually scaled and then added together and a threshold trigger set for the summed result.

Another variation on how a trigger condition can be set up is to have the trigger condition vary or be changed based on some other contaminant. For example, a trigger condition could be varied based on occupancy, if no one is in the space, the trigger conditions for some contaminants might be raised slightly. The trigger level could then be lowered when some one is detected or determined in some way to be in the space through, for example, occupancy sensor 27A in Room 20A, a card access system, or other means such as the detection of changes in CO2 in the space. There could also be manual local or remote override changes to the trigger levels, based on for example, an increased or decreased concern about the contaminants in the lab. Alternatively, the levels could be changed automatically by the signal processing controller 130, 210, 530, or 420 of FIG. 1,2,4, or 6 respectively, some other system such as the building automation or building control system 180, or a critical environment airflow control system.

Finally, any number of different logical or Boolean combinations of sensed contaminant values or sensor signal pattern conditions acting on any number of sensed contaminants affected by any other set of conditions or acted upon by other systems can be used to trigger a need for increased dilution ventilation by increasing dilution ventilation command 31.

There are a vast number of control techniques that may be used to generate command 31 in order to vary the amount of ventilation within the monitored critical environment 20 in order to dilute the sensed contaminant sufficiently to prevent the concentration of the airborne contaminant from exceeding a specific level. Any method that one may use, from a standpoint of control logic or algorithm, whether it be an open or closed loop strategy involving continuous or discontinuous control functions, fuzzy logic, proportional-integral-derivative functions, feed-forward functions, adaptive control, or other techniques known to those skilled in the art of control system design, are considered to be aspects of this invention.

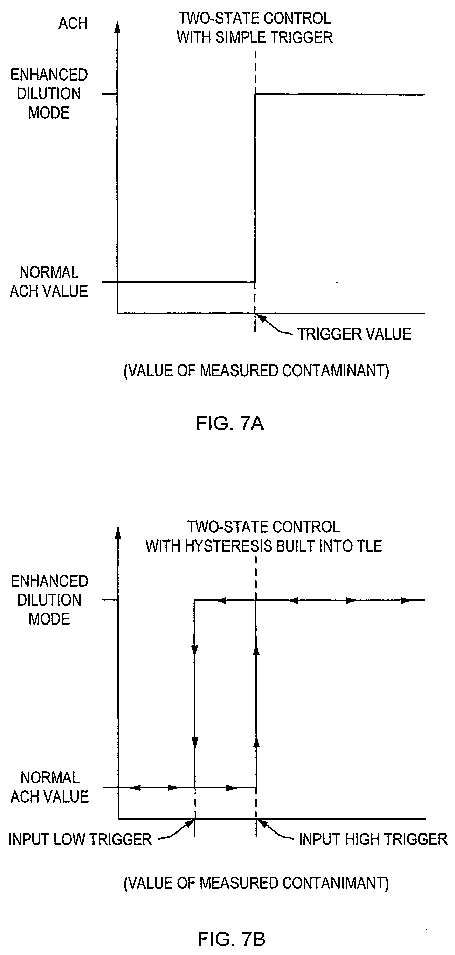

FIG. 7A illustrates one possible scenario of steady-state levels associated with command 31 when signal processing controller 130 is configured to provide a two-state control function such that command 31 is increased to an enhanced dilution mode level from a normal level or ACH (air changes per hour) value when a sensed contaminant from critical environment 20 transitions above an established trigger value. Conversely, when the value of the sensed contaminant transitions from a level that's above the trigger value to one below that value, command 31 will drop back to its normal steady state ACH value. FIG. 7A makes no reference to the time response of command 31 as it transitions from the normal ACH value to the Enhanced Dilution mode and vice versa, as this is a function of the particular control technique used to make such a transition while ensuring that stability is maintained within the system. As an embodiment of this invention the two-state approach of FIG. 7A can be acceptable for use in many applications. However, in some cases the system stability realized with the simple switching mechanism depicted by FIG. 7A will benefit by including provisions to prevent command 31 from oscillating.

As an embodiment of this invention, when command 31 is transitioned from the normal ACH value (3-4 ACH, for example) to the enhanced dilution mode (10-15 ACH, for example), command 31 will be latched or become fixed at that higher value, so that following the transition if the measured contaminant drops below the triggered value the air change rate will remain high. Such an approach may be accompanied by some form of notification mechanism from the Building Control System 180, or the sampling system 100, 300, 400, or via the internet connection 171, or from the air flow controller 30 or some other component of the system that airflow controller 30 connects to, which will alert maintenance personnel or other staff that the trigger value has been exceeded so that signal processing controller may be manually reset.

As an alternate embodiment, instead of latching command 31 when the value of the sensed contaminant exceeds an established trigger value, one may apply a hysteresis function as shown in FIG. 7B which depicts another scenario of steady-state levels associated with command 31, in which two different triggers or transition points are provided (input low trigger and input high trigger). Here the input high trigger is used when the command 31 is at a level corresponding to the normal ACH value, while the input low trigger is used when the command 31 is at a level corresponding to the enhanced dilution mode.

An exemplary type of control approach for dilution ventilation command signals 31 is a three state control approach. Unlike the previously mentioned control approach, which had two output levels such as a high level, typically for a purge, and a low normal operating level, this approach has three output levels. A typical application for these three levels would be the same two levels mentioned previously with an intermediate level added that is not for spills (an extreme transgression in the levels of a sensed contaminant) but for controlling more moderate levels of sensed contaminants that are desired to be lowered. For example, if a level of between 1 PPM and 10 PPM from the TVOC detector is sensed, the system would increment up a moderate level, say from a minimum level of 3 ACH to a level of 6 ACH's. However if the TVOC detector sensed levels above 10 PPM, then the system would go into a purge mode with perhaps 10 to 15 ACH's of dilution ventilation. This approach limits energy consumption for moderate contaminant levels and reduces the chance that if multiple rooms are at this moderate level, that the total system airflow capacity of the building will be exceeded by too many rooms being commanded to maximum air change rate (ACH) value. Another benefit of a three or other multiple level approach (or of a VAV approach as well) is that it lessens the chance of realizing an unstable condition where the room airflow can vary up and down due to a steady release of contaminants that alternately is purged to a low value and then slowly builds back up as the system alternately increases and overshoots and then decreases and undershoots the desired dilution airflow command level by an amount that exceeds what is required for a stable operating condition.

The three state control approach can be extended beyond three output states to any number of output states for dilution ventilation command signals 31 to provide different levels of dilution ventilation for a space. Finally any of the approaches to use multiple sensed signals such as from the shared sensors 120 and or a local room sensors 25A can as mentioned previously for the two state approach, also be used for the three or other multiple state control approaches.

An exemplary type of control approach for dilution ventilation command signals 31 is a variable air volume or VAV approach. In this approach, once the sensed contaminant signals reach some trigger level or match some signal pattern, the dilution ventilation command signal 31 can increase in a continuous manner from a minimum level which would match the minimum state output of the two or multiple state approach, all the way up to a maximum level that would correspond to the maximum level of the two state or multiple state approach. This effectively "infinite state" approach would as mentioned with the previous control approaches work with one or more sensed signals such as from the shared sensors 120 and or a local room sensors 25A that could be combined in any manner. One difference with this approach, however, is the need for a "trigger' signal that has a continuous output that is related to the command signal 31. This trigger signal can be formed from one or a plurality of sensed signals such as from the shared sensors 120 and or a local room sensor 25A as has been described previously.

A linear or non linear relationship can be established between this trigger signal and the command signal 31. For example with a linear relationship an offset and simple scale or gain factor can be used as well as a minimum and maximum clamp so that as the trigger signal increases above the minimum command signal value, the command signal 31 will increase as well until it hits the maximum allowed command signal value. One of the reasons to use a VAV approach is to create a closed loop control of the IEQ within the monitored space so as to prevent an oscillating control pattern that might be generated in some situations by a two state approach. With the VAV approach an increased ventilation level could be maintained between the minimum and maximum command signal 31 levels without an oscillating command level, particularly where there is a roughly constant level of contaminant emission. This approach could be used to regulate the level of an air contaminant such as a TVOC, particulate, or other at a certain setpoint rather than drive it to a minimum level that could prove to be costly in terms of the energy expense of running at high ventilation for extended periods. This approach could be appropriate when the contaminant is not a particularly hazardous one and can be set to be maintained at a level that would not create a health impact such as particles.

Alternatively this VAV approach could be used for another purpose with CO2 levels being sensed and used to set a given minimum ventilation rate related to the occupancy or number of people in the space such as given amount of outside air or cfm per person to meet certain building codes and guidelines such as those referred to in ASHRAE standard 62-2004 versus to control to a given contaminant level. This can be done since there is a fixed relationship between the level of CO2 in a space compared to the amount of people in that space divided by the amount of outside air introduced into the space. This type of control is sometimes referred to as demand control ventilation and has been used in office environments to allow outside air rates in facilities that use recirculated or return air to approximately track occupancy levels to a level such as for example 15 to 20 cfm per person. In this control approach CO2 is used as a proxy for directly measuring the cfm per person ventilation level due to the fixed amount of CO2 exhaled by people, approximately .01 CFM per person for a person doing light office work, providing a means to effectively measure the number of people in a space divided by the outside air introduced into that space.

In a VAV contaminant control approach where the contaminant is controlled to a set point value, alternatively, instead of only one signal, multiple of air contaminant signals may be combined or added, using methods similar to those mentioned earlier, to generate a single mixed or blended contaminant signal that can then itself be controlled to a setpoint value.

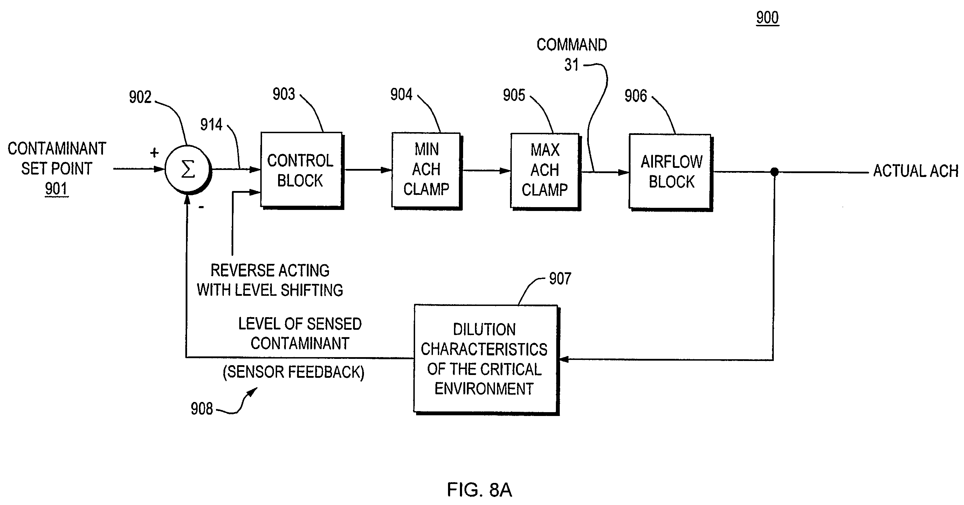

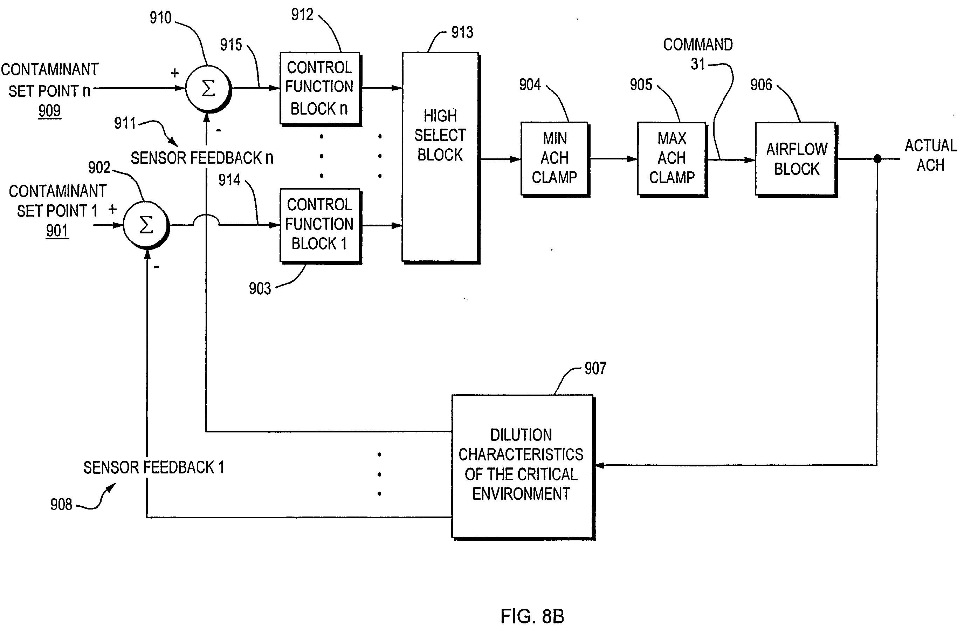

FIGs. 8A and 8B show another embodiment which is a generalized view of a closed loop system 900 used to provide dilution ventilation control by varying the air change rate within a critical environment, such as 20, in a continuous (or VAV) fashion within prescribed limits in order to prevent the level of a sensed contaminant, such as TVOC's for example, from exceeding a prescribed value. Here, sensor feedback 908 is subtracted from contaminant set point 901, which represents the level of the sensed contaminant that system 900 is to control to, in order to (by error stage 902) create error signal 914. Error signal 914 is acted upon by control block 903 in order to create a term that is bounded by Min ACH Clamp block 904 and Max ACH clamp 905 in order to yield command 31, which is the command to air flow block 906, which is composed of air flow controller 30 and the exhaust and supply flow (42 and 52) that it controls. Also depicted in FIG. 8A is block 907, which represents the dilution characteristics of the critical environment. For those who are familiar with the art of control system design, 907 represents the transfer characteristics of the environment which in this case defines how the air change rate of the environment under control relates to the value of the sensed contaminant 908. Here, error stage 902, reverse acting control block 903, Min ACH Clamp 904, and Max ACH clamp 905 may be implemented within signal processing controller 130, or within Building Control System 180.

Control block 903 may be implemented using any of a large number of control strategies known to those who are skilled in the art of control system design and may as an example include any combination of proportional control, proportional-integral control, proportional-integral-derivative control, feed forward techniques, adaptive and predictive control, and fuzzy logic strategies. One of the essential elements of control block 903 is that it provide the necessary reverse acting and level-shifting functions so that it may properly act upon error signal 914 (given the subtractive logic shown for error stage 902) in order to create a command term 31 which will yield an increase in the critical environment's air change rate at least for the condition where the sensor feedback 908 exceeds the contaminant set point 901. (Alternatively, the logic of 902 could be altered so that 901 is subtracted for 908.) As an example, contaminant setpoint 901 may be set to 1.5 ppm and the sensed contaminant may be, for example, TVOC's (using, for example a photo-ionization detector-or PID sensor-). Control block 903 will be configured so that when sensor feedback 908 is less than setpoint 901 the output of 903 will be less than or equal to the minimum clamp value established by minimum ACH clamp block 904.904 is a "high-select" block in that it will compare the value of the output of 903 to some minimum clamp value (4 ACH, for example) and present the larger of the two values to the next block 905. For example, if the output of 903 is 2 ACH and the minimum clamp value set in 904 is 4 ACH, the output of 904 will be 4 ACH. The output of 904 is presented to Max ACH clamp 905 which provides a "low-select" function in that it will compare the value of the output of 904 to a prescribed "max clamp" value (12 ACH, for example) and output the smaller of the two to air flow block 906. The way the system 900 works is that if there is some sudden increase in the level of the sensed contaminant (due to a spill in a laboratory, for example) above the contaminant setpoint 901 (set to 1.5 ppm TVOC's for example) the control block will (within the limitations of max clamp 905 set to 12 ACH, for example) increase command 31 to the value necessary to limit the TVOC concentration within the controlled environment to 1.5 ppm. In practice, set point 901 can be set to a value less than the TLV for the contaminant to be sensed to insure that sustained concentrations will be limited to a steady-state value that is safe. Alternatively, contaminant set point 901 may have a dynamic value that adjusts based on the persistence of the contaminant monitored by 908.

FIG. 8B illustrates an alternate embodiment of system 900 that provides the same control functions as FIG. 8A, but for any number "n" of contaminants. With this approach, a dedicated error stage 902 and control function block 903 are provided for each sensed contaminant (1 through "n"), with the nth sensed contaminant's set point shown as signal 909 going to error stage 910 which has an output 915 that is processed by function block 912. The outputs from each control block, such as from control blocks 903 to 912, are presented to high select block 913, which passes the largest of the control terms from the control blocks to airflow block 906 as command signal 31. Using this approach, one can provide dilution ventilation control to an environment such as 20 based on a number of contaminants, such as TVOC's, particles, and a host of other contaminants with individual setpoints such as 901 to 909 for each monitored contaminant.

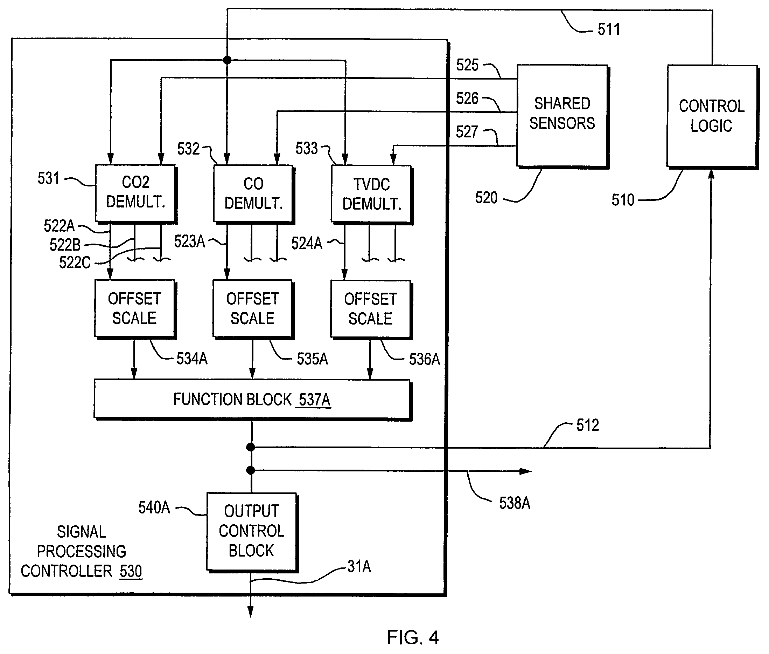

An implementation of a portion of the signal processing logic of the signal processing controller block 130 in FIG. 1, or of block 210 in FIG. 2 is shown in signal processing controller block 530 in FIG. 4. In this diagram the control functions can be implemented in analog or digital logic or be implemented with computer software or a firmware program or any combination of these. In FIG. 4, shared sensors 520 create one or a multiple of output signals or variables shown for example in the diagram as sensor signals 525, 526, and 527 representing the outputs of individual sensors CO2, CO, and TVOC's respectively. Although FIG. 4 illustrates the use of these three sensors, any number or type of sensors can be used. Since the sensors are being multiplexed with the air samples from multiple rooms, three in this example, the individual or "virtual" sensor signals for a given room corresponding to, as mentioned previously, a sensor signal or represented software variable for a given air contaminant in that room or area must be de-multiplexed from the signal stream of that contaminant. This is done within signal processing controller 530 by the de-multiplexers 531, 532 and 533 that de-multiplex the CO2, CO, and TVOC sensor signals respectively using the control signals 511 from the control logic block 510. Block 510 corresponds to control logic block 110 in FIG. 1 as well as part of signal processing controller block 210 and part of control logic block 310A, B, and C in FIG. 2. The output of the de-multiplexing blocks 531, 532, and 533 are individual or "virtual" sensor signals or software variables that represent the sensed contaminants or other air characteristics or air quality parameters for rooms 20A, B and C. For example, signals 522A, B and C represent the signals or variables for the sensed CO2 levels in rooms 20A, 20B and 20C, respectively.

These virtual sensor signals will typically have a value representing the last de-multiplexed value that will be held constant at that level until the next sampling of the corresponding location for that signal. At this point the signal will change value to equal the new de-multiplexed value. This transition of state from one de-multiplexed value to the next de-multiplexed value can occur either as a rapid or approximately step change in signal or it may occur gradually in a ramped manner lasting from several seconds in time up to many minutes depending on the desired properties of the virtual signal, what is being controlled with that signal, and how often the location is being sampled. A preferred approach would be to have a gradual change of value occurring over between 5 and 60 seconds.

If we again focus on the variables for Room 20A, then the signals for CO2, CO, and TVOC are 522A, 523A, and 524A respectively. As mentioned previously these individual or virtual sensor signals 522A, 523A, and 524A can then be modified with an offset and scale factor block 534A, 535A, and 536A respectively as needed or some other control function can then be applied. These modified signals from blocks 534A, 535A, and 536A are then acted upon by function block 537A. This is the block that may add these signals together, take the higher of the signals, apply threshold value or signal pattern trigger functions to the signals individually or as a group, or apply some other approach to combine or use these signals as mentioned previously. The result of block 537A after it has applied appropriate trigger criteria, some form of non-linear or linear scale and offset criteria, control loop gain function, or some other control or Boolean logic is to create a two state, three or multiple state, or a VAV dilution ventilation command signal. Finally, this command signal or control variable may then be outputted to a building control system or to another system as either a digital signal or variable such as signal 538A or as an airflow command signal or software variable such as the dilution ventilation airflow command signal 31A created by output block 540A and used as an input to room 20A's critical environments airflow control block 30A.

One other function that may be implemented within Function block 537A is a time delay or ramp function. For example, when a threshold value is exceeded, then the output of function block 537A which will become ventilation command signal 31A could be increased to it's maximum or purge value that might correspond for example to a room air change level of between 10 to 16 ACH's. This increase in value can occur instantly or may be commanded to be a gradual ramp by function block 537A. Such a ramp or slowly increasing signal could occur over the span of a minute or more. This might be done so as to not make an objectionably rapid increase in the room's flow level or cause problems with the control system trying to keep up with a rapidly changing signal that could cause a pressurization problem if the supply and exhaust control devices can not keep up with the changing signal. Similarly, when the ventilation command signal is meant to drop from a higher level such as 10 ACH down to a lower or minimum level such as 4 ACH, the function block 537A could create a slow ramp that gradually decreases the output signal 31A over some period of time such as one minute or more.

Similarly these increasing or decreasing ramps or gradual changes in level could be made linear, with constantly increasing or decreasing rates or made non-linear such as with an exponentially changing rate so the ramp could start faster and gradually slow down or conversely start slowly and gradually increase its rate of change in value until the signal hits it final value. These ramps could also be at different rates based on whether the signal is increasing or decreasing. For example, it may be advantageous to rapidly increase the dilution ventilation of a room by rapidly increasing the dilution ventilation command 31 if a spill or large increase in the contaminant level in the room is detected. However, it may also be helpful to have a slow ramp downward; perhaps taking 5 to 15 minutes to gradually come down in dilution ventilation flow to make sure that the contaminant is removed even to a level below the threshold of detection.

In an alternative to ramping the changing flow over a large signal range, it may, for the same reasons mentioned above, be desirable to change not just the rate of change of the dilution ventilation command 31 created by the signal, processing block 530 and function block 537A within it, but also the amount of the step change possible based on a change in the sensed air contaminants such as from the shared de-multiplexed sensor signals 522A, 523A, and or 524A. In other words, rather than allow a full slew from the minimum dilution rate to the maximum dilution rate from one air sample measurement, it may be desirable to limit the maximum step change in dilution ventilation airflow. For example a maximum step change size could be set for an increase in airflow representing four ACHs in a possible range from a minimum of four ACH to a maximum of sixteen ACH. With the maximum step size set for example for four ACH, it would take three successive air samples to have air contaminant values in excess of the trigger values to boost the dilution command 31 from the minimum to it's maximum value. Similarly, if the maximum reduction was also limited to a flow rate equal to four AC it would take three successive measurements of the critical environment's air contaminants to be below the trigger value for the dilution command level to drop from a level corresponding to sixteen ACHs down to four ACH.

In a manner similar to the ramp approach mentioned above, the increasing and decreasing step heights may be of different sizes. For example, to respond quickly to a spill there may be no limit or a larger limit for an upward or increasing change in dilution ventilation command 31. However, to ensure a large amount of dilution to very low levels and reduce the possibility of an oscillation if the source is not a spill, but a continuous emission, it may be advantageous to have a smaller decreasing step change size to hold the dilution ventilation at a higher level for longer periods so it takes several air sample cycles to fully reduce the ventilation level to its minimum level.

Another means to set the step heights or possibly the ramp rates is based on the level of detected contaminants or their rate of change. If a large value of contaminant and or a rapid rise in its level is detected since the last sample or recent samples, it may be advantageous to use different step change heights or ramp rates. For example in a spill, where there is a sudden increase to a large contaminant value, it may be prudent to immediately index the dilution command 31 to its maximum value. Smaller or more gradual increases in value could be set for smaller steps. On the other hand a sharp downward increase might not change the downward step level in order to keep the ventilation higher to better clean the air. Alternatively, for energy saving reasons and or if there happens to be many brief upward excursions of contaminant levels that may not be hazardous, it may be more beneficial, if the contaminant level has just rapidly dropped to below the trigger level to quickly drop the ventilation command 31 to its minimum level. As such, it may also be beneficial to have different step or output characteristics associated with each air contaminant. As a result, the output control characteristics would be different based on which air contaminant(s) triggered the need for more dilution ventilation.

Output signals of the signal processing controller block 530 may also be used to change the sampling sequence based on the detection of a spill or a level of contaminant that is of interest to more closely observe. In this alternate approach the sequencing of air samples into the shared sensors from the critical environments 20 may be altered through signal processing controller block output signal 512 that is used by control logic block 510 to modify the sampling sequence on a potentially temporary basis during the period of a detected event of interest in a particular space 20. Based on seeing the control signal or software variable 512 increase in value to some higher trigger level or exhibit some signal pattern such as a rapid rise in amplitude, the control logic block 510 might increase the frequency of the air sampling of the space where the event was detected. Alternatively or additionally, the areas around the affected space may be quickly sampled next or sampled at a higher frequency as well to look for a spread of the contaminant to other spaces. In the context of this invention a rapid rise in amplitude can be defined as a sudden increase in value to a level such as many times larger than the normal trigger level in less than 5 minutes such as that seen due to a spill of a volatile organic compound.

This change in sampling or control sequence can be implemented with the sampling system of either FIG. 1 or FIG. 2. If the latter system was being used, the detection of the event would be most likely carried out by the signal processing controller block 210 and the change in sequencing carried out by control logic blocks 310A, 310B, 310C and 310D.

Another change in control sequence that could be implemented if an event of some type is detected in a space or several spaces would be to change the sampling sequence by adding air sampling of several spaces at once to measure a mixed sample of several rooms. This could be implemented for example, by turning on one or more solenoids at once to gather a mixed sample of affected areas or of multiple areas nearby the affected area to rapidly look for potential spillage into other areas. This would be implemented in the same manner as mentioned above but would involve turning on multiple solenoid valves such as for example solenoids 161, 162, 263, and 164 in FIG. 1 or solenoids 361A, 362A, 363A, and 361B in FIG. 2.

With reference to FIG. 2, this diagram refers to another preferred embodiment of the present invention directed to dynamic control of dilution ventilation in one-pass, critical environments using a networked air sampling system such as one similar to that described in U.S. Patent No. 6,125,710. This sampling system has many of the functions and is similar to the system indicated in FIG. 1 with the main difference being that the solenoid switches and some of the controls are distributed throughout the building vs. being located in one central unit. As a result, central sampling unit 100 shown in FIG. 1 is effectively replaced by sensor and control unit 200, along with distributed air and data routers 300A, 300B, 300C, and 300D. The control of the sequencing of the system and the signal processing functions are handled by signal processing controller block 210. This block 210 carries out the functions of blocks 510 and 530 in FIG. 4 that have been described previously. The shared sensor block 220 carries out the same function as block 520 of FIG. 4 and block 120 of FIG. 1.

Blocks 300A, B, C and D are air and data routers that house the solenoid valves 361A, 362A, 363A, 361B, 362B, 361C and 361D as well as potentially some analog or digital input and output capabilities that are contained in Input/Output blocks 320A and 320B. As an example, air sampling location 23A is connected via tubing or air transport conduit 24A to solenoid 362A that is part of air and data router 300A. This tubing or air transport media 24A along with 44A, 14, 44B, 54B, 24C and 64 was described earlier except that the air transport conduit may also have associated with it some additional electrical conductors for the purpose of adding networked data communication, low voltage power, signal wires and other potential functions as described in U.S. Patent Application Serial No. 10/948,767, filed on September 23,2004 entitled, "TUBING FOR TRANSPORTING AIR SAMPLES IN AN AIR MONITORING SYSTEM", as well as U.S. Patent Application Serial No. 11/149,941 filed on June 10,2005 and entitled, "AIR MONITORING SYSTEM HAVING TUBING WITH AN ELECTRICALLY CONDUCTIVE INNER SURFACE FOR TRANSPORTING AIR SAMPLES". Adding these conductors enables local sensors to be conveniently and cost effectively added to the system.

For example, sampling location 23A, as well as the other sampling locations 43A, 43B, 53B, 24C and 63, could also contain a local temperature sensor to sense the room or duct temperature. The signal from this temperature sensor or from other sensors such as humidity, or other air characteristics can be sent to the air data router 300 as a digital data communications signal though a data communication cable such as a twisted pair, twisted shielded pair, fiber optic cable or other digital data communications media. Alternatively, the sensor information could be sent to the router 300 via an analog signal through one or more signal conductors as an analog voltage or current signal. This analog signal can then be converted to a digital signal by the I/O block 320A or 320B in the router 300A or 300B respectively.

These I/O blocks 320A and 320B can also monitor other air contaminants or signal inputs that are not associated with an air-sampling inlet yet would have a data communications cable, analog signal cable or other connection to the I/O block. An example of these sensors is Room Sensor 25A which could be a temperature sensor, an air contaminant sensor or other type of sensor such as a light, differential pressure, air velocity or other building sensor, as well as the occupancy sensor 27B, occupancy switch 28C, or emergency exhaust switch 81. Of the latter sensors or room switches, an occupancy sensor is defined in the context of this invention as a sensor that can detect the presence of people in a space through infra red energy, motion, card access, or other means, whereas an occupancy switch is defined in the context of this invention as a room switch such as a manually operated light switch or other type of room switch operated by the occupant when they enter or leave the space. A room switch in the context of this invention is defined as some type of switch that may be for example electrical, mechanical, photonic, or pneumatic that is located in or near the critical environment that can be manually operated to signal a change in state to a system connected to it. An emergency exhaust switch is defined as a room switch such as an electrical wall switch that can be thrown or actuated by the occupant when an emergency event has happened such as a fire, spill or explosion. The emergency exhaust switch may affect some outcome such as to provide maximum dilution ventilation to the space and or potentially provide a containment action by increasing the negative offset of the space or it may be for monitoring only. This room switch as well as some others may for convenience of sharing wiring be located in the same room location and possibly in the same enclosure as the air sampling pickup. Other types of room switches or sensors could also be connected to the I/O blocks 320 of the air and data routers 300.

Within the air data routers 300, the output of multiple solenoid valves can be manifolded together with manifold 390A and B. These manifolds plus the outputs of individual solenoid valves such as 361C in air and data router 300C or solenoid 361D in router 300D are connected together with tubing or air transport conduit 202 to transport air samples to shared sensors 220 in the multipoint air sampling unit 200 as moved by vacuum source 140. The control of the air and data routers as well as the communication of digital sensed air characteristic and contaminant data from the I/O blocks within the routers or from the local sensors in the spaces back to the multipoint air sampling unit 200 is through data communications cable 201. The air transport media 202 can be constructed using the same materials mentioned previously for tubing 24A and other connections from the spaces 20 to the routers 300. The data communications cable 201 can be made with any commonly used data communications media such as twisted pair, shielded twisted pair, fiber optics cable or other. Additionally in a preferred embodiment the air transport media 202 and the data communications media 201 can be combined into one structured cable as was described for the connections between the rooms 20 and the routers 300.

As in FIG. 1 the multipoint air sampling unit 200 also connects to the Internet 170 to send information about the critical environments to a password protected website for review by the occupants or facility personnel. Again as in FIG. 1 the multipoint sampling unit 200 can also interface to and send data back and forth through data communications media 181 with the facility's building control or management system 180. This can be done directly or through one of many interface protocols such as BacNet, Lon by Echelon, XML, OPC, or others.

In addition to the air and data routers 300 that can accept sensed input signals from the spaces 20 and provide signal outputs 31 and 32 to help control the rooms 20, the building control system 180 can also be used to accept various sensor input signals such as 29C from occupancy switch 28C and signal 82 from emergency exhaust switch 81. This information can be used by the building control system directly for control and also communicated back to the multipoint air sampling system 200. The building control system 180 can also provide control signals to help control the airflow in rooms 20 as shown by signals 31C, and 32C to the critical environments airflow control block 30C using sensor information from the multiplexed air sampling system 100 or 200 and potentially locally sensed signals, room switch information, as well as other building information.

FIG. 3 illustrates a more detailed diagram of one of the critical spaces and some of the airflow control and feedback devices and signals used therein. In addition to the components that have been described previously, this diagram also shows the room exhaust airflow sensing and control device or devices 41 and room exhaust airflow control signal 47 as well as room exhaust feedback signal 48. Also included is supply airflow sensing and control device or devices 51 and supply airflow control signal 57 and supply airflow feedback signal 58. A new device that has been shown is local temperature sensor 91 that communicates through cable 92 to a temperature controller 90. This temperature controller could be part of building control system 180, a stand-alone system, or part of a separate system that controls the airflow in a critical environment. Such a control system that includes for example special exhaust, room exhaust, and supply airflow controller devices 71, 41, and 51 respectively of FIG. 3 as well as the critical environment airflow controller 30 and controls at least room pressurization by maintaining either a given room pressure or volume offset is referred to in the context of this invention as a critical environment airflow control system which may also in some cases be referred to as a laboratory airflow control system. The purpose of temperature controller 90 is to provide temperature control which can include sending a thermal load or temperature command 93 to the critical environment airflow controller 30 to increase or decrease the supply airflow into space 20. The temperature control 90 may also control a reheat coil to increase the temperature of the supply air fed into the space 20 or perimeter heating coils in space 20 for further means of temperature control.

Another element not shown in FIG. 1,2, and 6 is the addition of a special exhaust airflow control and sensing device 71. This control device is connected to a special exhaust duct 70. Special exhaust device 72 could be one of many special exhaust devices such as a laboratory fume hood, snorkel exhaust, canopy hood, chemical storage cabinet, bio-safety cabinet, animal cage exhaust, or other device that exhausts air from the critical environment. Typically the flow of these devices is either fixed, two state or variable based on some aspect of the device. For example the flow through a laboratory fume hood can be made variable and proportional to the size of the fume hood sash opening to maintain a constant face velocity. This type of control can be seen in FIG. 3 where the special exhaust device 72 controls the special exhaust airflow control device 71 through airflow control signal 77. The special exhaust feedback or sensed airflow signal 78 is sent to the airflow controller block 30 along with potentially one or more other special exhaust air flow feedback signals illustrated for example by feedback signal 88.

FIG. 5 is an exemplary embodiment of the control diagram for the critical environment airflow controller 30. The supply airflow within a one-pass critical environment space is set by the higher of either the makeup air required by the space's special exhaust flows, the room's supply airflow requirement to meet the temperature command or the requirements for dilution ventilation in the space. This is implemented as shown in FIG. 5 by first summing any and all special exhaust feedback signals such as flow signals 78 and 88 by summing block 33. This totalized special flow exhaust feedback signal is then provided as one input into the high select signal comparator 34. Block 34 acts to take the highest of the three signals provided to it, passing which ever of the three signals is highest at any given time. The next input into high select block 34 is the temperature command 93 for varying supply flow which is then scaled and offset as needed in scaling block 38 to put it on the same scale factor as the other two airflow command signals inputs, such as certain number of cfm per volt for an analog voltage signal or scaled directly into a given set of units such as cfm or liters per second for a software or firmware variable representing airflow. The third signal is the dilution ventilation command signal 31 which is generated with the assistance of the multipoint air sampling system, the discrete local sensor system of FIG. 6, or the building control system 180 and is again scaled and offset as needed by scaling block 39 to put this command on the same scale factor as the other signals.

The command 57 for the supply airflow control device 51 is further shown created by taking the output of the high select comparator block 34 and subtracting offset signal 32 from it by subtraction block 37. The room offset airflow command 32 could be a fixed offset such as 10% of the maximum supply or exhaust cfm, or it could be a signal that varies in a two state, multi-state, or VAV fashion. The purpose of this offset airflow is to create a typically slight negative pressure for the room, although in some applications the offset airflow polarity can be flipped to instead create a net positive pressure in the space vs. the corridor and or other spaces. An exemplary application of the room offset airflow command 32 being a two state control signal is for signal 32 to be a value such as 10% of the maximum supply volume for normal room operation. However, when a spill or other emergency condition is detected such as a fire or smoke release via some sensor, alarm system, or manually with an emergency exhaust switch 81, the room offset airflow can be increased from its normal value by the sampling system, building control system, or the system of FIG. 6. Increasing the offset airflow to a potentially much higher value will reduce the supply airflow volume so as to create a large negative offset airflow for the room to provide a measure of increased containment to prevent the spread of the spill vapors or smoke into other spaces.

Finally FIG. 5 shows an embodiment of how command 47 for the room exhaust airflow control device is created by first starting with the supply flow feedback signal 58. The sum of the special exhaust feedback signals 78 and 88 is subtracted from the supply flow feedback signal 58 and added to the room offset airflow command 32. The resultant signal is the room exhaust command signal 47 that is used to set and control the flow of the room exhaust airflow control device 41.

FIG. 6 shows yet another facility monitoring system embodiment of the invention that uses only individual space or duct sensors located in the spaces or ducts to be monitored with no centralized sensing of the embodiments of FIG. 1 or 2. This embodiment can also combine and use the outputs of a plurality of room sensors such as 425A and 427A or others located in room 20A or in the room exhaust duct 40B of room 20B for dynamically varying the dilution ventilation of a one-pass critical environment 20 such as a laboratory or a vivarium. These one or more sensor outputs from the same space or sensor outputs from multiple locations can be combined as mentioned previously or used in a differential manner.

Describing the embodiment of FIG. 6 in more detail, room 20A contains two local or room sensors 425A and 427A. These sensors are connected via cables 426A and 428A respectively to I/O block 430A of the data acquisition and controller block 400A. These cables could be of many different media depending on the output of the sensor. For example, if these or other sensors have current or voltage outputs, analog signal wires could be used for the cables. If the output of the sensors 425A and 427A, or any other sensor, is a digital signal, then typically a twisted pair or shielded twisted pair would be used. If the output was of a digital optical or light signal then a fiber optic cable could be used for these and other local room sensors in FIG. 6.

Room sensors 425A and 427A could be one of many different sensors. For example, they could be a particle counter and a TVOC sensor or else they could be two or more of any of many other types of sensors as mentioned previously such as CO, CO2, ozone, radon, other toxic gases, ammonia, humidity, dew point temperature, light, differential pressure, etc.

Additionally, room 20B in FIG. 6 shows the use of local duct sensors such as individual sensors 443B and 445B mounted in the room exhaust duct 40B as well as local duct sensors 543B and 545B mounted in supply duct 50B. These four sensors are shown connected into I/O block 430B of data acquisition and controller block 400B through cables 444B, 446B, 454B, and 456B respectively. Similarly, room 20C shows the use of room sensors 425C and 427C that are connected to I/O block 430C of data acquisition and controller block 400C through cables 426C, and 428C. I/O block 430C also is monitoring duct sensors 463 and 465 that are mounted in an outside air duct to measure outdoor air conditions. These sensors 463 and 465 are connected to I/O block 430C through cables 464 and 466 respectively. Any of the sensors shown in FIG. 6 could be used to sense any one or more of the many air contaminants mentioned above or any other type of air contaminant, air characteristic, or building parameter of interest that can be sensed. Additionally, although not shown in FIG. 6, any of the room switches or room sensors shown in FIG. 1 or 2 such as occupancy switch 28C, emergency exhaust switch 81, occupancy sensor 27B, etc. can also be used with the embodiment of FIG. 6 by connecting these switches or sensors into one or more of the inputs of the I/O blocks 430.

In FIG. 6 the data acquisition and controller blocks 400A, 400B and 400C also contain control logic blocks 410A, 410B and 410C respectively. These control logic blocks are used to control the functioning and logic of the data acquisition and controller blocks 400 as well as help communicate and interface through communications cable media 401 with the other data acquisition and controller blocks and or with another building system such as the building control system 180. The data communications media 401 as well as 201, 181, and 171 can be defined in the context of this invention as a data network or communications cable which is part of some form of digital data communications network implemented for example with Ethernet or RS385 cable that runs a communications protocol such as BACnet, Lonworks, or a building controls or other building communications protocol such as Johnson Controls' Metasys N1 or N2 bus. Alternatively, an IP or Internet Protocol could be used.

The I/O block 430A like block 320A in FIG. 2 plus signal processing block 420A are also used to create analog or digital airflow control signals 31A and 32A for room 20A. In room 20B, the signal processing controller block 420B also generates a dilution ventilation airflow control signal 31B as an input into critical environment airflow controller 30B, however in this example, the controller 30B is a networked control device and receives all of its control and feedback signals via communications network 401. For this embodiment the airflow control signal 31B is in the form of a software variable or other form of digital information that is addressed to and received by the critical environments controller 30B. This type of networked control command 31B could likewise be employed in the embodiments of FIG. 1 or 2 using for example the digital communications media 201 of FIG. 2 or the building controls communications media 181 of either FIG. 1 or 2.

In a similar manner any of the airflow control devices, air contaminant sensors, or controllers described in FIG. 1, 2, and 6 could be networked digital devices whereby the control, feedback, and sensor signals could be digital information communicated between the devices via a networked communications systems such as a private LAN or local area network such as Ethernet or Arcnet, or even via a public communications network such as the Internet.

FIG. 6 also indicates how a critical environment airflow controller such as 30C can receive its control signals 31C and or 32C through the building control system 180 that is in communication with the data acquisition and controllers 400. Alternatively, all the data acquisition and controller functions indicated in FIG. 6 may be implemented and performed by building control system 180 without need for a separate data acquisition and control system such as indicated by separate blocks 400. In this latter case the controllers indicated by 400 would be implemented by building control system controllers or through other control or networked devices with control inputs and outputs of the type commonly manufactured and used by building control companies such as for example Johnson Controls with their Metasys system, Honeywell with their Alerton subsidiary's native BACnet system BACtalk, or Siemens with their Apogee system.

The signal processing controller blocks 420 or similar blocks implemented with the building control system 180 are used to combine the outputs of multiple air contaminant sensors using the same approaches mentioned earlier for the embodiments of FIG. 1 and 2. Similarly the dilution ventilation control signal 31 and the offset airflow control signals 32 can be created using the same methods mentioned earlier and can be of an output type such as two or three state or VAV as mentioned before for the systems of FIG. 1 and 2. Additionally any of the control or sensing approaches, or control inputs or outputs mentioned in FIG. 1, 2 or 6 can be applied to the systems or approaches of the other figures. Similarly these same approaches or systems can be applied to a facility monitoring system embodiment similar to that of either FIG. 1 or 2 that is implemented not with a multipoint air sampling system but instead using a fiber optic light packet sampling and sensing system such as described in US Patent # 6,252,689 and referred to in this patent as a networked photonic sampling system.

Using the systems of FIG. 1,2, or 6, or the networked photonic sampling system, there are several beneficial control implementations and methods that can be implemented to solve problems that occur when trying to vary the dilution ventilation in a one-pass critical environment. For example, the outdoor air that is being brought into the building may become slightly or significantly contaminated by one or more air contaminants. Such contaminants could include carbon monoxide from auto or truck exhaust or from re-entrainment of furnace or boiler exhaust, high levels of outdoor particulates, TVOC's that could be re-entrained from fume hood or other special exhaust stacks, or other outdoor sources of contaminants. If these contaminants are not filtered out and pass into the supply air that is being fed into the labs it could trigger the dilution ventilation controls to increase both the room exhaust and supply air flows. Similarly, the increase in supply air contaminants may not be high enough to trigger increased supply air flow commands by itself, but added to existing contaminant levels in the room it may make the system overly sensitive to low or moderate contaminant levels originating from within the room itself. Both of these problems can produce potentially runaway results since the control action of increasing supply air which contains air contaminants only serves to increase the level of contaminant within the room. This can drive the supply airflow levels even higher until no matter whether a two state, three state, or VAV approach is used the supply airflow into the room will eventually be commanded to its maximum level if the outdoor air or supply system contamination is high enough. Since the supply system airflow potentially feeds many rooms, potentially all of these rooms could be pushed to their maximum flows. This could result in the airflow capacity of the supply and or room exhaust system being exceeded with resultant reductions of flow into and out of the critical environment spaces and potential loss of pressurization levels of these spaces vs. the corridor or other rooms. If the special exhaust devices are also exhausted by the room exhaust fans, then these devices may lose capture and containment of hazardous fumes or vapors.

One exemplary control approach to solve this problem is to use a differential measurement technique. In this approach an outside air or supply air measurement is subtracted from room air measurements to create differential measurements of the various air contaminants of interest vs. either outside air or the supply air. Thus, if the outside or supply air has an increase in particles, CO, TVOC's, etc., the quality of the room air will be evaluated against sources of contaminants in the room only since the effect of the supply air sources will be subtracted out. Effectively, we are concerned here not with the absolute quality of the room air but whether it is being made worse by sources in the room or space only, since increasing the dilution air will not make the room cleaner if the dilution air is the source of the contaminant.

For example, as mentioned previously, we first start with air contaminant measurements of the air in space 20A using for example room sampling location 23A, room exhaust air duct sampling location 43A, and or room sensor 25A in FIG. 1 and 2, or room sensors 425A, 427A, and or room exhaust duct sensor 443A of FIG. 6. In this exemplary approach a measurement of the air contaminants is next made of either the outside air using air sampling location 63 in FIG. 1 or 2 or air contaminant sensors 463 and 465 of FIG. 6 in outside air intake duct 60 or the supply air using air sampling location 53B in FIG. 1 or 2 or supply airflow duct sensors 543B and 545B in supply airflow duct 50B. If the spaces are receiving 100% outside air directly from outdoors with no return air then a measurement of outside air from within the outside air duct 60 going into the supply air handler will provide accurate results for at least gas or VOC measurements. For at least particle measurements, however, the measurement must be taken after the air filters and fan systems such as at a location downstream of them such as the supply duct locations mentioned above. If return air from other areas is mixed with the outside air to produce the supply air, then the use of a downstream supply duct airflow measurement is also necessary with a location at least after where the outside air and return air become well mixed. The use of only one supply or outside air duct measurement should be sufficient for all the spaces fed from a single air handler or main supply duct since all the supply air flowing into these spaces from the same air system should have similar characteristics and contaminants.

Next each pair of air contaminant measurements (space air and outside or supply air) is turned into a set of differential measurements by subtracting the outside or supply air contaminant measurement from the space air contaminant measurement. An example of an embodiment to perform this is the subtraction block 35 of FIG. 5 where a supply or outside air measurement of for example TVOC's would be applied to the minus (-) input of the subtraction block and the room or room exhaust duct air contaminant measurement of TVOC's would then be applied to the positive (+) input. The output would then be the differential measurement of TVOC's for that space. Other methods of subtracting these air contaminant measurements for software variables in a computerized control system for example or for other implementations would be known to those well skilled in the art.

The individual differential air contaminant measurements would then be treated in the same manner described previously for the non-differential room air measurements and thus would be used, for example, individually or combined and then compared or analyzed by signal processing controller block 130, 210, 530 or 420 of FIG 1,2,4 or 6 respectively to create signals 31 and 32 that would be used to vary the supply and exhaust airflows of space 20.

The air sampling embodiments of FIG. 1 and 2 are preferred embodiments for this differential measurement control concept since the measurement of the supply or outside air and the space air measurement can be performed with the same sensor within a reasonably short period of time such as 5 to 30 minutes. As a result many sensor errors are eliminated since they cancel out when subtracting the two measurements. Consequently, very accurate differential measurements can be made even when the increase in contaminants in the room although important is relatively small compared to a potentially high source level of outside air contaminants. As a result these high outdoor background levels do not substantially decrease the resolution or accuracy of the measurement of the effects of any contaminant sources within the critical environment spaces.

Another control approach that can be used with the implementation of FIG. 1, 2 or 6 relates to a situation where a high level of supply or outside air contaminant may be present, yet the differential room air signal mentioned previously indicates that there are not substantive sources of contaminants in the space. In this situation the absolute level of contaminants in the space may be high enough to trigger an increased dilution level, but the differential signal correctly indicates that increasing the supply air is not appropriate. In this situation, since the source of the contaminant is the supply air, it may be advantageous to reduce the supply air until the outside or source air contains a lower level of contaminants.

One embodiment of this control approach consists of making one or more air contaminant measurements in the supply duct 50B or outside air intake duct 60 as mentioned previously. These one or more contaminant measurements can then be combined or used individually and then compared or analyzed by signal processing controller block 130, 210, 530 or 420 of FIG 1, 2, 4 or 6 respectively to determine if these signals exceed appropriate trigger levels such as those used for the critical environment spaces 20. If these trigger levels or appropriate trigger conditions are met, then blocks 130,210 or 420 can be used to reduce the supply flow by one of several approaches. For example, in FIG. 3 the temperature control output 93 of the Temperature control block 90 can be completely overridden and effectively disabled by a command output from signal processing controller blocks 130,210 or 420 so that the supply flow will become controlled by the higher of either just the makeup requirements of the special exhaust devices or the flow commanded by the dilution ventilation command 31 which would be reduced to a low level.

Another control approach that can be used with the implementation of FIG. 1, 2 or 6 relates to a situation where a high level of contaminants may be present around the room or space of interest particularly from a space such as the corridor which is positive to the room of interest and from which the room's offset airflow is drawn. In this situation, it may be desirable to create a differential signal for each contaminant of interest as was mentioned previously for outside or supply air. In this case, for example, the measured air contaminants from room 20 would be subtracted from the respective air contaminant measurements taken from corridor 10 or from an anteroom, or other space which provides at least a portion of room 20's offset airflow 21. When this differential signal shows a high level it indicates that the source of contaminants detected is in the room 20 not in the corridor 10 or other anteroom. This is important since in the case of someone cleaning the corridor with a cleaning agent that gives off VOC's, these VOC's will be pulled into all the rooms 20A, 20B and 20C that feed from the corridor 10. As such if the absolute level of contaminants or even the differential level of contaminants vs. outside air or the supply air is high in the rooms 20 due to the VOC's from the corridor it would throw all these rooms into a high level of ventilation. Although this may be acceptable, it may also cause problems with airflow capacity due to potentially all the rooms going to a high level of dilution ventilation. To prevent this airflow shortage condition from occurring, the differential level of these rooms can be checked vs the corridor or equivalently the absolute level of the corridor can be checked. Assuming the corridor is positive to the spaces of interest, if either the corridor level is high in any of the air contaminants of concern, or the level of the room is high whereas the differential level of the room compared to the corridor if low, then the correct control action may be to increase the level of ventilation in the corridor, but not to increase the level of ventilation in the room or only to partially increase this level to some intermediate level. Another means of implementing this control strategy is to only increase the room 20's dilution ventilation command when both the differential signal of the room to the supply (or outside air) is high and the differential signal of the room 20 to the corridor 10 (or other offset airflow source area) is also high.

Other strategies that could be implemented when these differential signals and corridor signals indicate the source of contamination is from the corridor 10 are to change the airflow direction to make the rooms positive vs. the corridor. For example there could be a source of smoke, VOC's or other contaminants either in the corridor or from another room that has in turn breached its containment and then contaminated the corridor. In these situations, the appropriate action could be to use the signal processing controller block 130, 210, 530 or 420 of FIG 1,2,4 or 6 to sense this condition as mentioned above and then use the room offset airflow control signals 32 to change the room 20's offset airflow 21 from into the rooms to out of the room. This would also require changing the corresponding offset airflow of the corridor from positive to negative so the combination of the rooms 20 and the corridor 10 remains balanced. In some cases the rooms 20 may already be positive to the corridor 10. In this case it may be advantageous to increase the level of positive offset airflow 21 to an even greater value to ensure better protection from the contaminants in the corridor 10. If the contaminant level in one of the rooms is higher than any other room, then that room may likely be the source of the contaminant. If that is the case then the signal processing controller block 130, 210, 530, or 420 of FIG 1,2,4, or 6 can be used to modify the offset airflow control signal 32 for that room 20 to make the room offset airflow 21 negative and to as high a level as is appropriate, while also modifying the corridor 10 offset airflow to the appropriate level. This concept of balancing offset airflows in a corridor 10 vs. the rooms 20 off from that corridor due to the need to change the offset airflows in a room 20 is described in U.S. Patent No. 5,545,086 entitled "Air Flow Control For Pressurized Room Facility".

## Claims

1. A dilution ventilation control system for use in a one-pass critical environment (20A, 20B, 20C) in which air is supplied to the critical environment using a supply airflow control device (51 A, 51B, 51C), and airflow out of the critical environment is exhausted using an exhaust airflow control device (41 A, 41 B, 41C), without any recirculation of exhausted air back to the critical environment (20A, 20B, 20C), the dilution ventilation control system comprising:
a facility monitoring system that uses at least one air contaminant sensor (120) to measure the level of an air contaminant in the critical environment (20A, 20B, 20C) and the level of the same air contaminant in the supply air, and generates output signals indicative of the level of the contaminant in the critical environment (20A, 20B, 20C) and the level of the contaminant in the supply air; and
a signal processing controller (130) responsive to the facility monitoring system output signals,
**characterised in that**:
the signal processing controller (130) determines a contaminant differential measurement equal to the difference between the measured contaminant level in the critical environment (20A, 20B, 20C) and the measured contaminant level in the supply air, and in response generates a dilution ventilation command signal that is provided to a critical environment airflow controller (30A,30B,30C) that uses said dilution ventilation command signal to control at least one of the supply airflow control device (51 A, 51B, 51C) and the exhaust airflow control device (41 A, 41B, 41C), thereby controlling the flow of at least one of the air supplied to the critical environment (20A, 20B, 20C) and the air exhausted from the critical environment (20A, 20B, 20C) so as to control the quantity of air changes per hour (ACH) in the critical environment (20A, 20B, 20C);
there is no re-circulation of the exhausted air back into the critical environment (20A, 20B, 20C);
the dilution ventilation command signal under normal conditions causes a minimum quantity of ACH in the critical environment (20A, 20B, 20C), and when a trigger event occurs the dilution ventilation command signal causes an increased quantity of ACH in the critical environment (20A, 20B, 20C); and
wherein a trigger event comprises at least one of:
(i) the contaminant level in the critical environment (20A, 20B, 20C) exceeding an input high trigger level; and
(ii) a rapid increase of the contaminant level in the critical environment (20A, 20B, 20C) even if the actual level is below the input high trigger level.

2. The dilution ventilation control system of claim 1 wherein the trigger event further comprises an input low contaminant trigger level that is less than the input high trigger level; and wherein after the quantity of ACH is increased it remains at the increased level until the contaminant level in the critical environment (20A, 20B, 20C) falls below the input low contaminant trigger level.

3. The dilution ventilation control system of claim 2 wherein the trigger event further comprises an input intermediate contaminant trigger level that is less than the input high trigger level and is greater than the input low contaminant trigger level, and when the intermediate contaminant trigger level is reached the quantity of ACH is increased to be greater than the minimum quantity of ACH and less than the increased quantity of ACH.

4. The dilution ventilation control system of any preceding claim wherein the critical environment (20A, 20B, 20C) comprises a special exhaust device (72).

5. The dilution ventilation control system of claim 4 wherein the amount of supply airflow is established to be the higher of:
(i) the makeup air required by outflow through the special exhaust device (72);
(ii) the supply airflow requirements of the critical environment (20A, 20B, 20C); and
(iii) the dilution ventilation requirement of the critical environment (20A, 20B, 20C).

6. The dilution ventilation control system of any preceding claim wherein under normal conditions the quantity of ACH in the critical environment (20A, 20B, 20C) is controlled to maintain a slight negative air pressure in the critical environment (20A, 20B, 20C) and when a trigger event occurs the airflow is controlled so as to increase the negative air pressure.

7. The dilution ventilation control system of any preceding claim wherein at least one of the air contaminant sensors comprises a total volatile organic compounds (TVOC) sensor.

8. The dilution ventilation control system of claim 7 wherein the TVOC sensor comprises a photo-ionization detector TVOC sensor.

9. The dilution ventilation control system of claim 8 wherein the photo-ionization detector TVOC sensor is calibrated with isobutylene and wherein the high trigger level is between about 0.3 to 5.0 ppm.

10. The dilution ventilation control system of any preceding claim wherein an airflow command signal is used to vary an offset airflow of the critical environment (20A, 20B, 20C).

11. The dilution ventilation control system of any preceding claim wherein an airflow command signal is used to increase both of the supply and exhaust air volumes when such airflow command signal commands a greater airflow than any other airflow command signals that are used to control the supply and exhaust air volumes.

12. The dilution ventilation control system of any preceding claim wherein both of the supply and exhaust air volumes are increased when a trigger event causes an increased quantity of ACH.

13. The dilution ventilation control system of any preceding claim wherein the signal processing controller (130) fixes an airflow command signal for a predetermined amount of time to increase at least one of the critical environment's supply and exhaust air volumes.

14. The dilution ventilation control system of any preceding claim wherein the signal processing controller (130) gradually adjusts the supply or exhaust air volumes over a time period greater than fifteen seconds.

15. The dilution ventilation control system of any preceding claim wherein the signal processing controller (130) fixes a limit on an amount to which the supply and exhaust air volumes may be changed during a given time period based on either a change in at least one of the sensed air contaminants or based on whether there is an increase in a value or a decrease in a value of the supply or exhaust air volumes.

16. The dilution ventilation control system of claim 15 wherein the limit varies dependent on which of the sensed air contaminants caused the need for additional dilution ventilation.

17. The dilution ventilation control system of any preceding claim wherein the facility monitoring system measures the level of two different air contaminants of the critical environment (20A, 20B, 20C) and generates first and second critical environment air contaminant measurement output signals; and wherein the signal processing controller (130), in response to the first and second critical environment air contaminant measurement output signals, generates an airflow command signal.

18. The dilution ventilation control system of claim 17 wherein the first and second critical environment air contaminant measurements are measured by a TVOC sensor and a particle sensor.

19. The dilution ventilation control system of claim 17 wherein the signal processing controller (130) generates an airflow command signal that changes value to increase at least one of the supply and exhaust air volumes when at least one of the first and second critical environment air contaminant measurements exceeds one or more predetermined threshold levels or approximately matches one or more predetermined signal patterns.

20. The dilution ventilation control system of claim 17 wherein the signal processing controller (130) generates an airflow command signal that changes value to increase at least one of the supply and exhaust air volumes when the first critical environment air contaminant measurement exceeds a threshold level that is a function of the second critical environment air contaminant measurement.

21. The dilution ventilation control system of any preceding claim further comprising one or more particle sensors, wherein the signal processing controller generates one or more of the airflow command signals based in further part on a level of particles in the critical environment sensed by the particle sensor.

## Patentansprüche

1. Verdünnungsbelüftungssteuersystem zur Verwendung in einer kritischen Einpassumgebung (20A, 20B, 20C), in der Luft der kritischen Umgebung mithilfe einer Zufuhrluftstrom-Steuervorrichtung (51A, 51B, 51C) zugeführt wird und der Luftstrom aus der kritischen Umgebung mithilfe einer Abluftstrom-Steuervorrichtung (41A, 41B, 41C) abgegeben wird, ohne dass die Abluft zurück in die kritische Umgebung (20A, 20B, 20C) zirkuliert, wobei das Verdünnungsbelüftungssteuersystem umfasst:
ein Anlagenüberwachungssystem, das mindestens einen Luftverschmutzungssensor (120) verwendet, um den Grad einer Luftverschmutzung in der kritischen Umgebung (20A, 20B, 20C) und den Grad der gleichen Luftverschmutzung in der Zufuhrluft zu messen, wobei der Sensor Ausgangssignale erzeugt, die den Grad der Verschmutzung in der kritischen Umgebung (20A, 20B, 20C) und den Grad der Verschmutzung in der Zufuhrluft angeben; und
eine Signalverarbeitungssteuerung (130), die auf die Ausgangssignale des Anlagenüberwachungssystems reagiert,
**dadurch gekennzeichnet, dass**:
die Signalverarbeitungssteuerung (130) eine Verschmutzungsdifferentialmessung bestimmt, die der Differenz zwischen dem gemessenen Verschmutzungsgrad in der kritischen Umgebung (20A, 20B, 20C) und dem gemessenen Verschmutzungsgrad in der Zufuhrluft entspricht und als Reaktion ein Verdünnungsbelüftungsbefehlssignal erzeugt, das einer Luftstromsteuerung der kritischen Umgebung (30A, 30B, 30C) bereitgestellt wird, die das Verdünnungsbelüftungsbefehlssignal verwendet, um mindestens entweder die Zufuhrluftstrom-Steuervorrichtung (51A, 51B, 51C) oder die Abluftstrom-Steuervorrichtung (41A, 41B, 41C) zu steuern, wodurch mindestens der Strom entweder der Luft, die der kritischen Umgebung (20A, 20B, 20C) zugeführt wird, oder der Luft, die aus der kritischen Umgebung (20A, 20B, 20C) abgeführt wird, gesteuert wird, um die Luftmengenveränderungen pro Stunde (ACH) in der kritischen Umgebung (20A, 20B, 20C) zu steuern;
wobei keine Rezirkulation der Abluft in die kritische Umgebung (20A, 20B, 20C) erfolgt;
wobei das Verdünnungsbelüftungsbefehlssignal unter normalen Bedingungen eine minimale ACH-Menge in der kritischen Umgebung (20A, 20B, 20C) herbeiführt und, wenn ein Auslöseereignis auftritt, das Verdünnungsbelüftungsbefehlssignal eine erhöhte ACH-Menge in der kritischen Umgebung (20A, 20B, 20C) herbeiführt; und
wobei ein Auslöseereignis mindestens eines von Folgendem umfasst:
(i) der Verschmutzungsgrad in der kritischen Umgebung (20A, 20B, 20C) überschreitet einen eingangs hohen Auslösegrad; und
(ii) eine rasche Zunahme des Verschmutzungsgrads in der kritischen Umgebung (20A, 20B, 20C), auch wenn der derzeitige Grad unterhalb des eingangs hohen Auslösegrads liegt.

2. Verdünnungsbelüftungssteuersystem nach Anspruch 1, wobei das Auslöseereignis ferner einen eingangs niedrigen Verschmutzungsauslösegrad umfasst, der geringer als der eingangs hohe Auslösegrad ist; und wobei nach Erhöhen der ACH-Menge diese bei dem erhöhten Grad bleibt, bis der Verschmutzungsgrad in der kritischen Umgebung (20A, 20B, 20C) unter den eingangs niedrigen Verschmutzungsauslösegrad fällt.

3. Verdünnungsbelüftungssteuersystem nach Anspruch 2, wobei das Auslöseereignis ferner einen eingangs mittleren Verschmutzungsauslösegrad umfasst, der niedriger als der eingangs hohe Auslösegrad ist und größer als der eingangs niedrige Verschmutzungsauslösegrad ist, und wobei, wenn der mittlere Verschmutzungsauslösegrad erreicht wird, die ACH-Menge erhöht wird, um höher als die minimale ACH-Menge und niedriger als die erhöhte ACH-Menge zu sein.

4. Verdünnungsbelüftungssteuersystem nach einem der vorhergehenden Ansprüche, wobei die kritische Umgebung (20A, 20B, 20C) eine spezielle Ablassvorrichtung (72) umfasst.

5. Verdünnungsbelüftungssteuersystem nach Anspruch 4, wobei die Menge des Zufuhrluftstroms höher erstellt wird als Folgende:
(i) die aufbereitete Luft, die durch den Abstrom durch die spezielle Ablassvorrichtung (72) erfordert wird;
(ii) die Anforderungen für den Zufuhrluftstrom der kritischen Umgebung (20A, 20B, 20C); und
(iii) die Anforderung für die Verdünnungsbelüftung der kritischen Umgebung (20A, 20B, 20C).

6. Verdünnungsbelüftungssteuersystem nach einem der vorhergehenden Ansprüche, wobei unter normalen Bedingungen die Menge an ACH in der kritischen Umgebung (20A, 20B, 20C) zum Beibehalten eines leichten Luftunterdrucks in der kritischen Umgebung (20A, 20B, 20C) gesteuert wird und, wenn ein Auslöseereignis auftritt, die Luftströmung gesteuert wird, um den Luftunterdruck zu erhöhen.

7. Verdünnungsbelüftungssteuersystem nach einem der vorhergehenden Ansprüche, wobei mindestens einer der Luftverschmutzungssensoren einen Sensor für alle flüchtigen organischen Verbindungen (TVOC) umfasst.

8. Verdünnungsbelüftungssteuersystem nach Anspruch 7, wobei der TVOC-Sensor einen Photoionisationsdetektor-TVOC-Sensor umfasst.

9. Verdünnungsbelüftungssteuersystem nach Anspruch 8, wobei der Photoionisationsdetektor-TVOC-Sensor mit Isobutylen kalibriert wird und wobei der hohe Auslösegrad zwischen etwa 0,3 bis 5,0 ppm beträgt.

10. Verdünnungsbelüftungssteuersystem nach einem der vorhergehenden Ansprüche, wobei ein Luftstrombefehlssignal zum Variieren eines Offset-Luftstroms der kritischen Umgebung (20A, 20B, 20C) verwendet wird.

11. Verdünnungsbelüftungssteuersystem nach einem der vorhergehenden Ansprüche, wobei ein Luftstrombefehlssignal zum Erhöhen sowohl des Zu- als auch des Abluftvolumens verwendet wird, wenn ein Luftstrombefehlssignal eine höhere Luftströmung als alle anderen Luftstrombefehlssignale anweist, die zum Steuern des Zu- und Abluftvolumens verwendet werden.

12. Verdünnungsbelüftungssteuersystem nach einem der vorhergehenden Ansprüche, wobei sowohl das Zu- als auch das Abluftvolumen erhöht werden, wenn ein Auslöseereignis eine erhöhte ACH-Menge herbeiführt.

13. Verdünnungsbelüftungssteuersystem nach einem der vorhergehenden Ansprüche, wobei die Signalverarbeitungssteuerung (130) ein Luftstrombefehlssignal für einen vorbestimmten Zeitraum zum Erhöhen mindestens entweder des Zu- oder Abluftvolumens der kritischen Umgebung erhöht.

14. Verdünnungsbelüftungssteuersystem nach einem der vorhergehenden Ansprüche, wobei die Signalverarbeitungssteuerung (130) das Zu- und Abluftvolumen über einen Zeitraum von mehr als fünfzehn Sekunden stufenweise anpasst.

15. Verdünnungsbelüftungssteuersystem nach einem der vorhergehenden Ansprüche, wobei die Signalverarbeitungssteuerung (130) eine Grenze bei einer Menge festlegt, zu der das Zu- und Abluftvolumen während eines vorgegebenen Zeitraums basierend entweder auf einer Veränderung in mindestens einer der erfassten Luftverschmutzungen oder basierend darauf, ob eine Zunahme in einem Wert oder eine Abnahme in einem Wert des Zu- und Abluftvolumens vorliegt, verändert werden kann.

16. Verdünnungsbelüftungssteuersystem nach Anspruch 15, wobei die Grenze je nachdem variiert, welches der erfassten Luftverschmutzungen den Bedarf an einer zusätzlichen Verdünnungsbelüftung herbeigeführt hat.

17. Verdünnungsbelüftungssteuersystem nach einem der vorhergehenden Ansprüche, wobei das Anlagenüberwachungssystem den Grad der zwei unterschiedlichen Luftverschmutzungen der kritischen Umgebung (20A, 20B, 20C) misst und erste und zweite Messausgangssignale für die Luftverschmutzung der kritischen Umgebung erzeugt; und wobei die Signalverarbeitungssteuerung (130) als Reaktion auf das erste und zweite Messausgangssignal für Luftverschmutzung der kritischen Umgebung ein Luftstrombefehlssignal erzeugt.

18. Verdünnungsbelüftungssteuersystem nach Anspruch 17, wobei die erste und die zweite Luftverschmutzungsmessung der kritischen Umgebung von einem TVOC-Sensor und einem Partikelsensor gemessen werden.

19. Verdünnungsbelüftungssteuersystem nach Anspruch 17, wobei die Signalverarbeitungssteuerung (130) ein Luftstrombefehlssignal erzeugt, das den Wert zum Erhöhen mindestens des Zu- oder Abluftvolumens erhöht, wenn mindestens entweder die erste oder die zweite Luftverschmutzungsmessung der kritischen Umgebung einen oder mehrere vorbestimmte Schwellengrade überschreitet oder etwa mit einem oder mehreren vorbestimmten Signalmustern übereinstimmt.

20. Verdünnungsbelüftungssteuersystem nach Anspruch 17, wobei die Signalverarbeitungssteuerung (130) ein Luftstrombefehlssignal erzeugt, das den Wert zum Erhöhen mindestens des Zu- oder Abluftvolumens erhöht, wenn die erste Luftverschmutzungsmessung der kritischen Umgebung einen vorbestimmten Schwellengrad überschreitet, der von der zweiten Luftverschmutzungsmessung der kritischen Umgebung abhängig ist.

21. Verdünnungsbelüftungssteuersystem nach einem der vorhergehenden Ansprüche, ferner umfassend einen oder mehrere Partikelsensoren, wobei die Signalverarbeitungssteuerung eines oder mehrere der Luftstrombefehlssignale erzeugt, die des Weiteren auf einem Grad der Partikel in der kritischen Umgebung basieren, die von dem Partikelsensor erfasst werden.

## Revendications

1. Système de commande de ventilation à dilution à utiliser dans un environnement critique mono-passe (20A, 20B, 20C), dans lequel de l'air est fourni à l'environnement critique en utilisant un dispositif de commande d'écoulement d'air d'alimentation (51A, 51B, 51C), et un écoulement d'air hors de l'environnement critique est évacué en utilisant un dispositif de commande d'écoulement d'air d'échappement (41A, 41B, 41C), sans aucune recirculation d'air évacué vers l'environnement critique (20A, 20B, 20C), le système de commande de ventilation à dilution comprenant:
un système de surveillance d'installation qui utilise au moins un capteur de contaminant de l'air (120) pour mesurer le niveau d'un contaminant de l'air dans l'environnement critique (20A, 20B, 20C) ainsi que le niveau du même contaminant de l'air dans l'air d'alimentation, et qui génère des signaux de sortie indicatifs du niveau du contaminant dans l'environnement critique (20A, 20B, 20C) ainsi que le niveau de contaminant dans l'air d'alimentation; et
un dispositif de commande de traitement de signal (130) sensible aux signaux de sortie du système de surveillance,
**caractérisé en ce que**:
le dispositif de commande de traitement de signal (130) détermine une mesure différentielle de contaminant qui est égale à la différente entre le niveau de contaminant mesuré dans l'environnement critique (20A, 20B, 20C) et le niveau de contaminant mesuré dans l'air d'alimentation, et génère en réponse un signal de commande de ventilation à dilution qui est transmis à un dispositif de commande d'écoulement d'air d'environnement critique (30A, 30B, 30C) qui utilise ledit signal de commande de ventilation à dilution pour commander au moins un parmi le dispositif de commande d'écoulement d'air d'alimentation (51A, 51B, 51C) et le dispositif de commande d'écoulement d'air d'échappement (41A, 41B, 41C), commandant de ce fait l'écoulement d'au moins un parmi l'air fourni à l'environnement critique (20A, 20B, 20C) et l'air évacué à partir de l'environnement critique (20A, 20B, 20C) de manière à commander la quantité de changements d'air par heure (ACH) dans l'environnement critique (20A, 20B, 20C);
il n'y a pas de recirculation de l'air évacué vers l'environnement critique (20A, 20B, 20C);
le signal de commande de ventilation à dilution dans des conditions normales entraîne une quantité minimum d'ACH dans l'environnement critique (20A, 20B, 20C), et lorsqu'il se produit un événement de déclenchement, le signal de commande de ventilation à dilution entraîne une quantité accrue d'ACH dans l'environnement critique (20A, 20B, 20C); et
dans lequel un événement de déclenchement comprend au moins un parmi:
(i) le niveau de contaminant dans l'environnement critique (20A, 20B, 20C) dépasse un niveau de déclenchement élevé d'entrée; et
(ii) une augmentation rapide du niveau de contaminant dans l'environnement critique (20A, 20B, 20C) même si le niveau réel se situe en dessous du niveau de déclenchement élevé d'entrée.

2. Système de commande de ventilation à dilution selon la revendication 1, dans lequel l'événement de déclenchement comprend en outre un niveau de déclenchement de contaminant bas d'entrée qui est inférieur au niveau de déclenchement élevé d'entrée; et dans lequel après que la quantité d'ACH ait été accrue, elle reste au niveau accru jusqu'à ce que le niveau de contaminant dans l'environnement critique (20A, 20B, 20C) chute en dessous du niveau de déclenchement de contaminant bas d'entrée.

3. Système de commande de ventilation à dilution selon la revendication 2, dans lequel l'événement de déclenchement comprend en outre un niveau de déclenchement de contaminant intermédiaire d'entrée qui est inférieur au niveau de déclenchement élevé d'entrée et qui est supérieur au niveau de déclenchement de contaminant bas d'entrée, et lorsque le niveau de déclenchement de contaminant intermédiaire est atteint, la quantité d'ACH est accrue de manière à être supérieure à la quantité minimum d'ACH et inférieure à la quantité accrue d'ACH.

4. Système de commande de ventilation à dilution selon l'une quelconque des revendications précédentes, dans lequel l'environnement critique (20A, 20B, 20C) comprend un dispositif d'échappement spécial (72).

5. Système de commande de ventilation à dilution selon la revendication 4, dans lequel la quantité d'écoulement d'air d'alimentation est établie de manière à être la plus élevée parmi:
(i) l'air d'appoint requis par l'écoulement de sortie à travers le dispositif d'échappement spécial (72) ;
(ii) les exigences d'écoulement d'air d'alimentation de l'environnement critique (20A, 20B, 20C); et
(iii) l'exigence de ventilation à dilution de l'environnement critique (20A, 20B, 20C).

6. Système de commande de ventilation à dilution selon l'une quelconque des revendications précédentes, dans lequel dans des conditions normales, la quantité d'ACH dans l'environnement critique (20A, 20B, 20C) est commandée de manière à maintenir un légère pression d'air négative dans l'environnement critique (20A, 20B, 20C), et lorsqu'il se produit un événement de déclenchement, l'écoulement d'air est commandé de manière à augmenter la pression d'air négative.

7. Système de commande de ventilation à dilution selon l'une quelconque des revendications précédentes, dans lequel au moins un des capteurs de contaminant de l'air comprend un capteur de composés organiques volatils totaux (TVOC).

8. Système de commande de ventilation à dilution selon la revendication 7, dans lequel le capteur de TVOC comprend un capteur de TVOC à détecteur par photo-ionisation.

9. Système de commande de ventilation à dilution selon la revendication 8, dans lequel le capteur de TVOC à détecteur par photo-ionisation est calibré avec de l'isobutylène, et dans lequel le niveau de déclenchement élevé est compris entre environ 0,3 ppm et 5,0 ppm.

10. Système de commande de ventilation à dilution selon l'une quelconque des revendications précédentes, dans lequel un signal de commande d'écoulement d'air est utilisé pour modifier un écoulement d'air décalé de l'environnement critique (20A, 20B, 20C).

11. Système de commande de ventilation à dilution selon l'une quelconque des revendications précédentes, dans lequel un signal de commande d'écoulement d'air est utilisé pour augmenter à la fois le volume d'air d'alimentation et le volume d'air d'échappement lorsque ce signal de commande d'écoulement d'air commande un écoulement d'air plus important que tous les autres signaux de commande d'écoulement d'air qui sont utilisés pour commander les volumes d'air d'alimentation et d'air d'échappement.

12. Système de commande de ventilation à dilution selon l'une quelconque des revendications précédentes, dans lequel le volume d'air d'alimentation et le volume d'air d'échappement sont tous les deux accrus lorsqu'un événement de déclenchement engendre une quantité d'ACH accrue.

13. Système de commande de ventilation à dilution selon l'une quelconque des revendications précédentes, dans lequel le dispositif de commande de traitement de signal (130) fixe un signal de commande d'écoulement d'air pour une quantité prédéterminée de temps pour augmenter au moins un des volumes d'air d'alimentation et d'air d'échappement de l'environnement critique.

14. Système de commande de ventilation à dilution selon l'une quelconque des revendications précédentes, dans lequel le dispositif de commande de traitement de signal (130) ajuste graduellement les volumes d'air d'alimentation et d'air d'échappement sur une période de temps qui est supérieure à quinze secondes.

15. Système de commande de ventilation à dilution selon l'une quelconque des revendications précédentes, dans lequel le dispositif de commande de traitement de signal (130) fixe une limite à une quantité de laquelle les volumes d'air d'alimentation et d'air d'échappement peuvent être modifiés pendant une période de temps donnée sur la base soit d'un changement dans au moins un des contaminants de l'air détectés, ou sur la base d'une augmentation d'une valeur ou d'une diminution d'une valeur des volumes d'air d'alimentation et d'air d'échappement.

16. Système de commande de ventilation à dilution selon la revendication 15, dans lequel la limite varie en fonction du contaminant de l'air détecté qui a entraîné la nécessité d'une ventilation à dilution supplémentaire.

17. Système de commande de ventilation à dilution selon l'une quelconque des revendications précédentes, dans lequel le système de surveillance d'installation mesure le niveau de deux contaminants de l'air différents de l'environnement critique (20A, 20B, 20C), et génère des premier et deuxième signaux de sortie de mesure de contaminants de l'air de l'environnement critique; et dans lequel le dispositif de commande de traitement de signal (130), en réponse aux premier et deuxième signaux de sortie de mesure de contaminant de l'air de l'environnement critique, génère un signal de commande d'écoulement d'air.

18. Système de commande de ventilation à dilution selon la revendication 17, dans lequel les première et deuxième mesures de contaminants de l'air de l'environnement critique sont mesurées par un capteur de TVOC et un capteur de particules.

19. Système de commande de ventilation à dilution selon la revendication 17, dans lequel le dispositif de commande de traitement de signal (130) génère un signal de commande d'écoulement d'air dont la valeur change pour augmenter au moins un des volumes d'air d'alimentation et d'air d'échappement lorsqu'au moins une des première et deuxième mesures de contaminants de l'air de l'environnement critique dépasse un ou plusieurs niveau(x) de seuil prédéterminé(s) ou coïncide approximativement avec un ou plusieurs motif(s) de signal prédéterminé(s).

20. Système de commande de ventilation à dilution selon la revendication 17, dans lequel le dispositif de commande de traitement de signal (130) génère un signal de commande d'écoulement d'air dont la valeur change pour augmenter au moins un des volumes d'air d'alimentation et d'air d'échappement lorsque la première mesure de contaminant de l'air de l'environnement critique dépasse un niveau de seuil qui est une fonction de la deuxième mesure de contaminant de l'air de l'environnement critique.

21. Système de commande de ventilation à dilution selon l'une quelconque des revendications précédentes, comprenant en outre un ou plusieurs capteur(s) de particules, dans lequel le dispositif de commande de traitement de signal génère un ou plusieurs signal (signaux) de commande d'écoulement d'air sur la base en outre en partie d'un niveau de particules dans l'environnement critique détecté par le capteur de particules.
